# EUROPEAN PATENT SPECIFICATION

(11) **EP 3 464 623 B1**
(45) Date of publication and mention of the grant of the patent: **01.09.2021**
(21) Application number: 17728514.5
(22) Date of filing: 01.06.2017
(51) Int. Cl.: C12Q 1/6886

(54) **ANDROGEN RECEPTOR SPLICE VARIANTS AND ANDROGEN DEPRIVATION THERAPY**
ANDROGENREZEPTORSPLEISSVARIANTEN UND ANDROGENDEPRIVATIONSTHERAPIE
VARIANTS D'ÉPISSAGE DU RÉCEPTEUR D'ANDROGÈNE ET THÉRAPIE PAR PRIVATION ANDROGÉNIQUE

(30) Priority: 02.06.2016 EP 16172764
(43) Date of publication of application: 10.04.2019
(73) Proprietor: Sint-Augustinus, 2610 Wilrijk (BE); Universiteit Antwerpen, 2000 Antwerpen (BE)
(72) Inventor: DIRIX, Luc, 2610 Wilrijk (BE); VAN LAERE, Steven, 2000 Antwerpen (BE); DE LAERE, Bram, 2000 Antwerpen (BE); HUGET, Philippe, 2610 Wilrijk (BE)
(74) Representative: Arnold & Siedsma
(86) International application number: PCT/EP2017/063329
(87) International publication number: WO 2017/207702

(56) References cited:
- WO-A1-2015/023710
- WO-A1-2015/065919
- WO-A2-2009/128936
- US-A1- 2014 107 180
- EMMA HÖRNBERG ET AL: "Expression of Androgen Receptor Splice Variants in Prostate Cancer Bone Metastases is Associated with Castration-Resistance and Short Survival", PLOS ONE, vol. 6, no. 4, 28 April 2011 (2011-04-28), page e19059, XP055205987, DOI: 10.1371/journal.pone.0019059
- K. E. WARE ET AL: "Biologic and clinical significance of androgen receptor variants in castration resistant prostate cancer", ENDOCRINE - RELATED CANCER, vol. 21, no. 4, 23 May 2014 (2014-05-23), pages T87-T103, XP055315528, GB ISSN: 1351-0088, DOI: 10.1530/ERC-13-0470
- P. A. WATSON ET AL: "Constitutively active androgen receptor splice variants expressed in castration-resistant prostate cancer require full-length androgen receptor", PROCEEDINGS OF THE NATIONAL ACADEMY OF SCIENCES, vol. 107, no. 39, 7 September 2010 (2010-09-07), pages 16759-16765, XP055266802, US ISSN: 0027-8424, DOI: 10.1073/pnas.1012443107
- Y. LI ET AL: "Androgen Receptor Splice Variants Mediate Enzalutamide Resistance in Castration-Resistant Prostate Cancer Cell Lines", CANCER RESEARCH, vol. 73, no. 2, 1 November 2012 (2012-11-01), pages 483-489, XP055238265, US ISSN: 0008-5472, DOI: 10.1158/0008-5472.CAN-12-3630

## Description

### FIELD OF THE INVENTION

The present invention provides androgen receptor splice variant AR-V5 in combination with circulating tumour cell (CTC) count as markers associated with resistance to androgen deprivation therapy, such as treatment with abiraterone acetate or enzalutamide. The present invention is especially useful in the field of oncology, including prostate cancer diagnosis, characterization, classification and treatment.

### BACKGROUND TO THE INVENTION

Prostate cancer is the most frequently diagnosed cancer in men, and is the fifth most common cause of cancer death worldwide. With 9.036 cases in 2011, prostate cancer is the most common cancer in Belgian men. Prostate cancer is an androgen receptor (AR)-dependent disease with the majority of patients demonstrating benefit from androgen deprivation therapy (ADT). However, eventually all patients with advanced disease progress towards castration-resistant prostate cancer (CRPC), which is associated with deterioration in quality of life, worse prognosis and a median survival of 18-24 months.

After castration or other means of ADT, and thus under low systemic testosterone conditions, prostate cancer cells are still at least partially dependent for their proliferation on the AR signaling pathway. Increased serum PSA levels upon castration-resistant prostate cancer indicate a reactivation of the AR pathway and acts as clinical proof of disease progression dependent on this pathway. Few therapeutic options are currently available for CRPC, yet a continued strategy of targeting the AR axis was shown to be useful, hence the development of several new hormonal therapies. Abiraterone acetate (a testosterone biosynthesis inhibitor) will have a beneficial effect in 29% and 62% of docetaxel-treated or chemotherapy-naïve patients, respectively (Nabhan et al., Annals of Oncology, 2011, 22:1948-1957; de Bono et al., Engl J Med, 2011, 364:1995-2005; Ryan et al., Engl J Med, 368:138-14). In patients treated with enzalutamide (an AR-antagonist) after taxane chemotherapy, 54% showed a reduction in serum PSA levels by 50% or more (Beer et al., Engl J Med, 2014, 371:424-433; Scher et al., Engl J Med, 367:1187-1197). Even though clinical responses are impressive, an important subgroup of CRPC patients remains unresponsive. Relatively little is known about the mechanisms behind non-response to the new hormonal therapies and chemotherapies.

In an effort to better understand abiraterone resistance, Mostaghel et al. (Clin Cancer Res 2011, 17:5913-5925) observed that treatment of CRPC xenografts with abiraterone led to higher expression of the AR splice variants AR-V7 and AR-V12 (termed AR^{V567es} by Mostaghel et al.). Li et al. (Cancer Res 2013, 73:483-489) demonstrated in vitro that cells expressing the same splice variants, AR-V7 and AR-V12, were resistant to enzalutamide. Selective knockdown of the expression of these splice variants inhibited androgen-independent growth and restored responsiveness to enzalutamide. Antonorakis et al. (N Engl J Med 2014, 371:1028-1038) showed that AR-V7 expression in circulating tumor cells (CTCs) from men with advanced prostate cancer was associated with resistance to enzalutamide and abiraterone.

WO2014047285 describes study results of the same AR splice variants, AR-V7 and AR-V12 (termed Arv5,6,7), as potential markers for taxane sensitivity. Interestingly, it was found that AR-V7 and AR-V12 react very differently in relation to taxane sensitivity. The inventors of WO2014047285 identified AR-V7 as being associated with taxane resistance, while AR-V12 was associated with taxane sensitivity. They suggest testing of both AR-V7 and AR-V12. If more of AR-V12 is present compared to AR-V7, the patient might benefit from taxane treatment. In contrast, if less AR-V12 is present compared to AR-V7, the inventors suggest that the patient may not benefit from taxane therapy. Furthermore, Ware et al. (Endocr Relat Cancer 2014, 21:T87-T103) and others previously suggested that AR-V1 would not be linked to resistance against ADT. These results show that different AR splice variants may display very different (even opposite) effects in drug resistance.

In addition to the splice variants AR-V7 and AR-V12, other AR-related drug resistance mediators have been evaluated. Another reoccurring androgen receptor splice variant, being associated with androgen deprivation therapy is AR-V1, but then typically in combination with AR-V7 (see for example WO2009/128936, WO2015023710 and WO2015/065919) and accordingly underscores that throughout the reported studies AR-V1 is not consistently linked with unresponsiveness to ADT, or at least in itself lacks the resolving power of differentiating all responders over the non-responders. Besides AR-V1, WO2014066864 and WO2014018926 shows that the F876L mutation in AR confers resistance to enzalutamide. WO2015/023710 also mentions that the count of circulating tumor cells CTCs can also be taken into account as a biomarker for tumor response and prognosis after treatment and that it can be combined with AR splice variants. However, there is no specific focus on AR-V5.

Despite the recent progress in the identification of markers for therapy resistance, AR-V7, AR-V12 and F876L cannot explain the resistance observed for second-generation CRPC therapies, such as enzalutamide and abiraterone, in all patients. Consequently, despite their clinical success, a significant number of CRPC patients that are non-responsive are still being treated with these expensive drugs and are exposed to their side-effects. Thus, there is an ongoing need to identify other markers for resistance to CRPC drugs, such as enzalutamide, abiraterone and taxanes.

### SUMMARY OF THE INVENTION

It has surprisingly been found that several splice variants of the androgen receptor, in particular AR-V1, AR-V2, AR-V3, AR-V5, AR-V9, AR-V10 and AR45, are associated with resistance against androgen deprivation therapy and with a reduced progression-free survival.

As apparent from the experimental part hereinafter, the present specification convincingly shows AR-V10's association with resistance against androgen deprivation therapy and with a reduced progression-free survival. It further shows, and contrary to the teaching in the field that in a multivariate analysis the androgen receptor splice variant ARV5 is a predictive marker for reduced progression-free survival of prostate cancer patients receiving ADT. AR-V5 accordingly allows to differentiate the responders over the non-responders to ADT, in particular when used in combination with CTC as tumor marker. Therefore, in a first aspect, the present invention provides a method for determining if a tumor will be resistant to androgen deprivation therapy (ADT), the method comprising determining a) the presence or absence of an androgen receptor splice variant consisting of AR-V5 in a circulating tumor cell (CTC) enriched fluid sample of said tumor; and b) detecting Circulating Tumor Cells in said sample wherein the presence of the AR-V5 splice variant and CTC indicates that the tumor will be resistant to androgen deprivation therapy. In a further embodiment, the present invention provides a method for determining if a patient will have a reduced chance to benefit from ADT, the method comprising determining a) the presence or absence of an androgen receptor splice variant consisting of AR-V5 in a circulating tumor cell (CTC) enriched fluid sample of said tumor; and b) detecting Circulating Tumor Cells in said sample wherein the presence of the AR-V5 splice variant and CTC indicates that the patient will have a reduced chance to benefit from ADT.

In principle, any of the AR splice variants described herein can be used in the methods not comprised in the present invention. In a particular embodiment not part of the invention, said AR splice variant is selected from the group consisting of AR-V1, AR-V2, AR-V3, AR-V5, AR-V9, AR-V10 and AR45, in particular AR-V1, AR-V2, AR-V5 and AR-V10; more in particular AR-V5 and AR-V10. Said variant detection may be combined with the detection of one or more other AR splice variants or AR mutations.

Another particular embodiment not part of the invention comprises the detection of at least one AR splice variant selected from the group consisting of AR-V1, AR-V2, AR-V5 and AR-V10 optionally in combination with one or more AR splice variants selected from the group consisting of AR-V3, AR-V7, AR-V9 and AR45; in particular AR-V3, AR-V7 and AR-V9; more in particular AR-V7. In a specific embodiment said AR splice variant is AR-V5; optionally in combination with one or more AR splice variants selected from the group consisting of AR-V1, AR-V2, AR-V3, AR-V7, AR-V9, AR-V10 and AR45; in particular AR-V3, AR-V7, AR-V9 and AR-V10; more in particular AR-V3, AR-V7 and AR-V9.

In a particular embodiment, determining the presence or absence of an AR splice variant comprises determining the expression level of said splice variant. A higher expression level of said splice variant indicates any of a reduced progression-free survival, a reduced chance of benefiting from ADT and an increased resistance to ADT.

Determining the presence or absence of an AR splice variant may be performed at the nucleic acid or protein level. In a particular embodiment, it is performed by determining the presence or absence or the expression level of the AR splice variant nucleic acid sequence in the sample. In a further embodiment, said determining comprises a PCR-based method. In a further embodiment, the method of the invention comprises the detection of multiple AR splice variants by multiplex PCR. In another embodiment, the present invention comprises nucleic acid sequencing. In another embodiment, said determining is a protein-based detection. In a further embodiment, the method of the invention comprises determining the presence or absence of an AR splice variant using a binding agent that specifically binds to the AR splice variant protein. Preferred binding agents include antibodies, nanobodies, aptamers, or fragments thereof.

In a particular embodiment, the androgen deprivation therapy comprises antiandrogen therapy. In a more particular embodiment, the antiandrogen therapy comprises administration of abiraterone acetate or enzalutamide. Therefore, in a further embodiment, the present invention indicates resistance against or a reduced chance of benefiting from abiraterone acetate.

In another particular embodiment, the tumor as described herein is a prostate tumor, in particular a prostate cancer, more in particular is a prostate cancer that has metastasized. In another embodiment, the tumor is a castration-resistant prostate cancer (CRPC), more in particular metastatic castration-resistant prostate cancer (mCRPC).

In a preferred embodiment, the present invention is applied on a biological fluid sample, also known as a liquid biopsy. Therefore, in a particular embodiment, the tumor sample is a biological fluid sample, in particular a blood sample or a sample derived therefrom. A suitable sample derived from a blood sample includes a plasma sample. In a particular embodiment, the method comprises determining the presence of an AR splice variant using cell-free tumor nucleic acids that are present in a biological fluid sample. In another particular embodiment, the presence is determined using circulating tumor cells. The present invention further comprises circulating tumor cell (CTC) enrichment.

The method of the invention comprises determining circulating tumor cell count, and the expression level AR-V5. In particular the methods of the invention comprise determining the presence or absence of the androgen receptor splice variant AR-V5 and the tumor marker CTC in a sample of said tumor; wherein the presence of AR-V5 and CTC indicates that the tumor will be resistant to androgen deprivation therapy, i.e. is prognostic for reduced progression-free survival of the patient. In an embodiment not part of the invention, the methods comprise determining the presence or absence of the androgen receptor splice variant ARV10; and the tumor marker CTC in a sample of said tumor; wherein the presence of ARV10 and CTC indicates that the tumor will be resistant to androgen deprivation therapy, i.e. is prognostic for reduced progression-free survival of the patient. In an even further particular embodiment not part of the invention, the methods comprise determining the presence or absence of the androgen receptor splice variant ARV15; and the tumor marker CTC in a sample of said tumor; wherein the presence of ARV5 and CTC indicates that the tumor will be resistant to androgen deprivation therapy, i.e. is prognostic for reduced progression-free survival of the patient.

In another embodiment, the present invention provides a computing system for predicting whether a tumor will be resistant to ADT. The computing system not part of the invention, may e.g. comprise
- a storage medium comprising a database structure for accepting androgen receptor splice variant information from a sample of a tumor, wherein the androgen splice variant is selected from the group consisting of AR-V1, AR-V2, AR-V5 and AR-V10; in particular AR-V5 and AR-V10; more in particular AR-V5 and
- a computer program product that, when executed on a processing engine,
   a) determines the presence or absence of an androgen receptor splice variant selected from the group consisting of AR-V1, AR-V2, AR-V5 and AR-V10; in particular AR-V5 and AR-V10: more in particular AR-V5 in the sample based on the androgen receptor splice variant information in the database structure, and
   b) indicates that the tumor will be resistant to androgen deprivation therapy if the presence of the androgen receptor splice variant has been determined in step a).

In another embodiment, not part of the invention, the present disclosure provides a computing system for predicting whether a tumor will be resistant to ADT. The computing system may e.g. comprise
- a storage medium comprising a database structure for accepting androgen receptor splice variant information from a sample of a tumor, wherein the androgen splice variant is selected from the group consisting of AR-V3, AR-V5, AR-V7, and AR-V45; in particular AR-V3, AR-V5, and AR-V7; more in particular AR-V5 and
- a computer program product that, when executed on a processing engine,
   a) determines the presence or absence of an androgen receptor splice variant selected from the group consisting of AR-V3, AR-V5, AR-V7, and AR-V45; in particular AR-V3, AR-V5, and AR-V7; more in particular AR-V5 in the sample based on the androgen receptor splice variant information in the database structure, and
   b) indicates that the tumor will be resistant to androgen deprivation therapy if the presence of the androgen receptor splice variant has been determined in step a).

The present invention provides a computing system for predicting whether a tumor will be resistant to ADT. The computing system comprises
- a storage medium comprising a database structure for accepting androgen receptor splice variant and tumor marker information from a sample of a tumor, wherein the androgen splice variant is AR-V5 and wherein the tumor marker is CTC; and
- a computer program product that, when executed on a processing engine,
   a) determines the presence or absence of AR-V5 in the sample based on the androgen receptor splice variant information in the database structure,
   b) determines the presence or absence of the CTC tumor marker; and
   c) indicates that the tumor will be resistant to androgen deprivation therapy if the presence of the androgen receptor splice variant has been determined in step a) and the presence of the CTC tumor marker has been determined in step b).

### BRIEF DESCRIPTION OF THE DRAWINGS

With specific reference now to the figures, it is stressed that the particulars shown are by way of example and for purposes of illustrative discussion of the different embodiments of the present invention only. They are presented in the cause of providing what is believed to be the most useful and readily description of the principles and conceptual aspects of the invention. In this regard no attempt is made to show structural details of the invention in more detail than is necessary for a fundamental understanding of the invention. The description taken with the drawings make apparent to those skilled in the art how the several forms of the invention may be embodied in practice.
**Fig. 1****: Kaplan meier plots patient groups that are negative or positive for a particular AR splice variant:** A: AR-V1, B: AR-V2, C: AR-V3, D: AR-V5, E: AR-V7, F: AR-V9, G: AR-V10, H: AR45. The X-axis displays the time in days, the Y-axis is the progression-free survival (PFS) probability.
**Fig. 2****: Kaplan-meier plots after removal of outlier patient:** A: AR-V2, B: AR-V3.
**Fig. 3****: AR splice variant status of cohort patients.** A: Patients have been grouped according to time of no longer clinically benefiting (NLCB): less than 6 months, between 6 and 12 months and more than 12 months. AR splice variant status is shown for AR-V7, AR-V1, AR-V2, AR-V5 and AR-V10. B: Number of AR-V2 transcripts per 1000 reads for patients in the cohort. C: Association between the number of AR-V2 transcripts per 1000 reads within groups with PFS less than 6 months and more than 12 months.
**Fig. 4****: AR splice variant status of cohort patients.** A: Amount of AR splice variants normalized per 1000 reads per circulating tumor cell. B: Kaplan-meier showing for AR splice variant negative and AR splice variant (i.e. any measured AR splice variant) positive patients.

### DETAILED DESCRIPTION OF THE INVENTION

Generally, nomenclatures utilized in connection with, and techniques of, cell and tissue culture, molecular biology, and protein and oligo- or polynucleotide chemistry and hybridization described herein are those well known and commonly used in the art. Standard techniques are used for recombinant DNA, oligonucleotide synthesis, and tissue culture and transformation (e.g., electroporation, lipofection). Enzymatic reactions and purification techniques are performed according to the manufacturer's specifications or as commonly accomplished in the art or as described herein. The foregoing techniques and procedures are generally performed according to conventional methods well known in the art and as described in various general and more specific references that are cited and discussed throughout the present specification. See e.g., Sambrook et al. Molecular Cloning: A Laboratory Manual (2d ed., Cold Spring Harbor Laboratory Press, Cold Spring Harbor, N.Y. (1989). The nomenclatures utilized in connection with, and the laboratory procedures and techniques of, analytical chemistry, synthetic organic chemistry, and medicinal and pharmaceutical chemistry described herein are those well known and commonly used in the art. Standard techniques are used for chemical syntheses, chemical analyses, pharmaceutical preparation, formulation, and delivery, and treatment of patients.

The present invention is described in separate particular and preferred embodiments. As is evident to the skilled person, it is envisaged that different embodiments may be combined unless the context clearly dictates otherwise.

The androgen receptor (AR), also termed the Dihydrotestosterone receptor (DHTR), has been sequenced and its protein sequence is available from several websites. For example, the full-length human androgen receptor protein sequence is available from the National Center for Biotechnology Information (NCBI) website with accession number P10275.2 (GI:113830) and referred-to herein as SEQ ID NO: 1. All positional information in relation to the AR protein, e.g. amino acid numbering, as used herein refers to said sequence unless explicitly mentioned. The sequence of the AR may vary from one person to another. In particular, the number of repetitive glutamine residues (amino acids 58-89 of SEQ ID NO: 1) and repetitive glycine residues (amino acids 446-472 of SEQ ID NO: 1) may increase or decrease somewhat. Thus, for example, a reference to the detection of an amino acid at a specific position number refers to the detection of the amino acid at the position corresponding to said specific amino acid position in SEQ ID NO: 1, but that position may be somewhat higher or lower in the actual AR (variant) protein being tested.

In addition to the protein sequence, nucleotide sequences are also available from the NCBI database. For example, a cDNA sequence for the full-length human AR is available under accession number M20132.1 (GI:178627), referred-to herein as SEQ ID NO: 2. All positional information in relation to the AR nucleic acid, e.g. nucleotide numbering, as used herein refers to said sequence unless explicitly mentioned. Similar to the protein sequence, the corresponding nucleotide number may be higher or lower in the actual AR sequence being tested. As also explained herein, corresponding nucleotides may be analyzed in the full-length AR gene.

The androgen receptor splice variants described herein are named according to the generally accepted AR splice variant naming scheme, which is for example described in Lu and Luo (Transl Androl Urol 2013, 3:178-186). The following table provides an overview of the protein and cDNA sequences of the AR spice variants as used herein.

| AR splice variant | protein sequence | cDNA sequence |
|---|---|---|
| AR-V1 | SEQ ID NO: 3 | SEQ ID NO: 4 |
| AR-V2 | SEQ ID NO: 5 | SEQ ID NO: 6 |
| AR-V3 | SEQ ID NO: 7 | SEQ ID NO: 8 |
| AR-V5 | SEQ ID NO: 9 | SEQ ID NO: 10 |
| AR-V7 | SEQ ID NO: 11 | SEQ ID NO: 12 |
| AR-V9 | SEQ ID NO: 13 | SEQ ID NO: 14 |
| AR-V10 | SEQ ID NO: 15 | SEQ ID NO: 16 |
| AR45 | SEQ ID NO: 17 | SEQ ID NO: 18 |

As described above, the AR sequence and, consequently, the AR splice variant sequence may contain small intersubject changes. Thus, detection of an AR splice variant within the meaning of this application means the detection of an AR splice variant with the general sequence build-up as described in Lu and Luo (2013), i.e. containing the same order of (partial) intron and exon blocks. In a particular embodiment, determining the presence or absence of an AR splice variant comprises detecting the presence or absence of a protein sequence having a sequence identity of at least 95% compared to SEQ ID NO: 3, SEQ ID NO: 5, SEQ ID NO: 7, SEQ ID NO: 9, SEQ ID NO: 13, SEQ ID NO: 15, or SEQ ID NO: 17. In another particular embodiment, said determining comprises detecting the presence or absence of a nucleic acid sequence encoding for said protein sequence. In yet another particular embodiment, determining the presence or absence of an AR splice variant comprises detecting the presence or absence of a nucleic acid sequence having a sequence identity of at least 95% compared to SEQ ID NO: 4, SEQ ID NO: 6, SEQ ID NO: 8, SEQ ID NO: 10, SEQ ID NO: 14, SEQ ID NO: 16, or SEQ ID NO: 17, or the RNA equivalent thereof. In particular embodiments, the sequence identity for said protein and/or nucleic acid sequences is at least 98%, more in particular at least 99%.

As described herein before, determining the presence of an AR splice variant can be performed by determining the presence or absence of the AR splice variant protein or a corresponding nucleic acid. As used herein, detection of a protein sequence can be performed directly, i.e. by determining if the protein itself is present, or indirectly, i.e. by determining if a nucleic acid (DNA, RNA, or cDNA) is present that encodes for said protein. In a preferred embodiment, determining the presence or absence of an AR splice variant comprises detecting the presence or absence of the corresponding nucleic acid in the sample. Of note, AR splice variants contain signature sequences, such as a particular intron and/or exon splicing or junction, and/or stop codons. To practice the present invention, it is not required to determine the full AR splice variant sequence, but it may be sufficient to determine the presence of such a signature sequence to determine that the corresponding AR splice variant is present or absent. A signature sequence for an AR splice variant as used herein refers to a nucleic acid sequence of at least 32 nucleotides or a protein sequence of at least 8 amino acids, wherein said sequence allows to differentiate said AR splice variant from other AR splice variants. In a particular embodiment, the signature sequences are the sequences that are amplified using the primer sequences of the examples. As an example, determining the presence or absence of an AR splice variant may comprise an amplification step wherein at least a part of the AR splice variant is amplified and detecting the presence of the amplified product using a specific hybridizing agent that specifically hybridizes to said AR splice variant (such as a hybridization probe) or by sequencing the amplified product. In a particular embodiment, determining the presence or absence of an AR splice variant comprises detection of the AR splice variant with a specifically hybridizing labeled probe. Determining the presence or absence of an AR splice variant may be performed according to the detection methods described for AR-V7 in WO2009128936, adapted where necessary to the detection of the specific variants described herein.

In a particular embodiment not part of the invention, AR-V1 presence or absence is determined by detecting the Ex3-CE1 fusion region, where Ex3 is preceded by exon 2 . In a more particular embodiment, it is determined by detecting the presence of a nucleic acid sequence having at least 95%, 96%, 97%, 98%, 99% percent sequence identity to SEQ ID NO: 19 or by detecting the presence of a nucleic acid sequence consisting of SEQ ID NO: 19. In another particular embodiment not part of the invention, AR-V2 presence or absence is determined by detecting the duplicated Ex3 (Ex3a-Ex3b) fusion region, followed by CE1. In a more particular embodiment, it is determined by detecting the presence of a nucleic acid sequence having at least 95%, 96%, 97%, 98%, 99% percent sequence identity to SEQ ID NO: 20 or by detecting the presence of a nucleic acid sequence consisting of SEQ ID NO: 20. In another particular embodiment not part of the invention, AR-V3 presence or absence is determined by detecting the Ex2-CE4 fusion region. In a more particular embodiment, it is determined by detecting the presence of a nucleic acid sequence having at least 95%, 96%, 97%, 98%, 99% percent sequence identity to SEQ ID NO: 21 or by detecting the presence of a nucleic acid sequence consisting of SEQ ID NO: 21. AR-V5 presence or absence is determined by detecting the Ex3-CE2 fusion region. In a more particular embodiment, it is determined by detecting the presence of a nucleic acid sequence having at least 95%, 96%, 97%, 98%, 99% percent sequence identity to SEQ ID NO: 22 or by detecting the presence of a nucleic acid sequence consisting of SEQ ID NO: 22. In another particular embodiment not part of the invention, AR-V7 presence or absence is determined by detecting the Ex3-CE3 fusion region. In a more particular embodiment, it is determined by detecting the presence of a nucleic acid sequence having at least 95%, 96%, 97%, 98%, 99% percent sequence identity to SEQ ID NO: 23 or by detecting the presence of a nucleic acid sequence consisting of SEQ ID NO: 23. In another particular embodiment not part of the invention, AR-V9 presence or absence is determined by detecting the Ex3-CE5 fusion region. In a more particular embodiment, it is determined by detecting the presence of a nucleic acid sequence having at least 95%, 96%, 97%, 98%, 99% percent sequence identity to SEQ ID NO: 24 or by detecting the presence of a nucleic acid sequence consisting of SEQ ID NO: 24. In another particular embodiment not part of the invention, AR-V10 presence or absence is determined by detecting the Ex3-3b fusion region, where 3b (or I3 (see Lu et al., 2015, Nat. Rev. Urol. 12:137-144) is a specific noncanonical splice site situated within CE3. In a more particular embodiment, it is determined by detecting the presence of a nucleic acid sequence having at least 95%, 96%, 97%, 98%, 99% percent sequence identity to SEQ ID NO: 25 or by detecting the presence of a nucleic acid sequence consisting of SEQ ID NO: 25. In another particular embodiment not part of the invention, AR45 presence or absence is determined by detecting the 1b-Ex2 fusion region. In a more particular embodiment, it is determined by detecting the presence of a nucleic acid sequence having at least 95%, 96%, 97%, 98%, 99% percent sequence identity to SEQ ID NO: 26 or by detecting the presence of a nucleic acid sequence consisting of SEQ ID NO: 26.

In another particular embodiment not part of the invention, the methods of the present invention comprise:
a) performing an amplification step comprising at least 10 amplification cycles with at least one primer set, wherein said amplification step produces amplicons that comprise at least 15 (in particular at least 20, more in particular at least 30) continuous nucleotides from any of SEQ ID NO: 19 to 26; and
b) a detection step comprising determining the presence or absence of the amplicons, thereby determining the presence or absence of the corresponding AR splice variant.

In a further embodiment, said detection step is performed using a hybridization probe that binds specifically to an amplicon. In another further embodiment, said detection step is performed by sequencing said amplicons.

Determining the presence or absence of an AR splice variant may comprise determining its presence if the AR splice level variant is detected above a predetermined threshold and determining the absence if the AR splice variant is detected below the predetermined threshold.

The term "sequence identity" means that two polynucleotide or amino acid sequences are identical (i.e., on a nucleotide-by-nucleotide or residue-by-residue basis) over the comparison window. The term "percentage of sequence identity" is calculated by comparing two optimally aligned sequences over the window of comparison, determining the number of positions at which the identical nucleic acid base (e.g., A, T, C, G, U, or I) or residue occurs in both sequences to yield the number of matched positions, dividing the number of matched positions by the total number of positions in the comparison window (i.e., the window size), and multiplying the result by 100 to yield the percentage of sequence identity. The terms "substantial identity" as used herein denotes a characteristic of a polynucleotide or amino acid sequence, wherein the polynucleotide or amino acid comprises a sequence that has at least 85 percent sequence identity, preferably at least 90 to 95 percent sequence identity, more usually at least 96, 97, 98, or 99 percent sequence identity as compared to a reference sequence over a comparison window of at least 18 nucleotide (6 amino acid) positions, frequently over a window of at least 24-48 nucleotide (8-16 amino acid) positions, wherein the percentage of sequence identity is calculated by comparing the reference sequence to the sequence which may include deletions or additions which total 20 percent or less of the reference sequence over the comparison window. The reference sequence may be a subset of a larger sequence. In preferred embodiments of the invention, sequence identity is determined for a window of at least 18 nucleotides (6 amino acids), in particular at least 24-48 nucleotides (8-16 amino acids). In a further embodiment, sequence identity is determined over the complete length of the sequences disclosed herein. At each instance, if a minimal sequence identity percentage is given, the invention also envisions the use of higher minimal sequence identities. In particular, at each instance, "at least 95% sequence identity" also entails a particular embodiment of "at least 97% sequence identity", "at least 99% sequence identity" and the identical sequence (i.e. having 100 % sequence identity).

The term "selectively hybridize" referred to herein means to detectably and specifically bind. Polynucleotides, oligonucleotides, and fragments thereof selectively hybridize to nucleic acid strands under hybridization and wash conditions that minimize appreciable amounts of detectable binding to nonspecific nucleic acids. High stringency conditions can be used to achieve selective hybridization conditions as known in the art and discussed herein.

As used herein, the terms "label" or "labeled" refers to incorporation of a detectable marker, e.g., by incorporation of a radiolabeled amino acid or attachment to a polypeptide of biotinyl moieties that can be detected by marked avidin (e.g., streptavidin containing a fluorescent marker or enzymatic activity that can be detected by optical or colorimetric methods). In certain situations, the label or marker can also be therapeutic. Various methods of labeling polypeptides and glycoproteins are known in the art and may be used. Examples of labels for polypeptides include, but are not limited to, the following: radioisotopes or radionuclides, fluorescent labels (e.g., FITC, rhodamine, lanthanide phosphors), enzymatic labels (e.g., horseradish peroxidase, beta-galactosidase, luciferase, alkaline phosphatase), chemiluminescent groups, biotinyl groups, and predetermined polypeptide epitopes recognized by a secondary reporter (e.g., leucine zipper pair sequences, binding sites for secondary antibodies, metal binding domains, epitope tags). In some embodiments, labels are attached by spacer arms of various lengths to reduce potential steric hindrance.

The term "specifically binds" refers to the ability of a specific binding agent to bind to a target with greater affinity than it binds to a non- target. In certain embodiments, specific binding refers to binding for a target with an affinity that is at least 10, 50, 100, 250, 500, or 1000 times greater than the affinity for a non-target. In certain embodiments, affinity is determined by an affinity ELISA assay. In certain embodiments, affinity is determined by a BIAcore assay. In certain embodiments, affinity is determined by a kinetic method. In certain embodiments, affinity is determined by an equilibrium/solution method. In certain embodiments, an antibody, antibody fragment or nanobody is said to specifically bind an antigen when the dissociation constant between the antibody, antibody fragment or nanobody and one or more of its recognized epitopes is <1 [mu]M, preferably < 100 nM and most preferably < 10 nM.

The term "antibody" refers to both an intact antibody and a antigen binding fragment thereof which competes with the intact antibody for specific binding. "Antigen binding fragment thereof refers to a portion or fragment of an intact antibody molecule, wherein the fragment retains the antigen-binding function. Binding fragments are produced by recombinant DNA techniques, or by enzymatic or chemical cleavage of intact antibodies such as by cleavage with papain. Binding fragments include Fab, Fab', F(ab')2, Fv, single-chain antibodies ("scFv"), Fd<1> and Fd fragments. Methods for producing the various fragments from monoclonal antibodies are well known to those skilled in the art (see, e.g., Pluckthun, 1992, Immunol. Rev. 130:151-188).

The terms "treat" or "treatment" refer to both therapeutic treatment and prophylactic or preventative measures, wherein the object is to prevent or slow down (lessen) an undesired physiological change or disorder, such as the development or spread of cancer. For purposes of this invention, beneficial or desired clinical results include, but are not limited to, alleviation of symptoms, diminishment of extent of disease, stabilized (i.e., not worsening) state of disease, delay or slowing of disease progression, amelioration or palliation of the disease state, improvement of the quality of life (especially by avoiding side effects from unnecessary therapy) and remission (whether partial or total). "Treatment" can also mean prolonging survival as compared to expected survival if not receiving treatment. Those in need of treatment include those already with the condition or disorder as well as those prone to have the condition or disorder or those in which the condition or disorder is to be prevented or its occurrence to be slowed down.

The term "responsive" as used herein means that a patient or tumor shows a complete response or a partial response after administering an agent, according to RECIST 1.1 (Response Evaluation Criteria in Solid Tumors, version 1.1) or PCWG3 (the Prostate Cancer Clinical Trials Working Group 3) criteria. The term "nonresponsive" or "resistance" as used herein means that a patient's disease shows progressive disease after administering an agent, according to accepted response criteria (RECIST 1.1 or PCWG3). RECIST 1.1 is described, e.g., in Eisenhauer et al., 2009, " New response evaluation criteria in solid tumours: Revised RECIST guideline (version 1.1)," EUROPEAN JOURNAL OF CANCER 45 (2009) 228-247; while PCWG3 is described e.g. in Scher et al., J Clin Oncol 2016; 34:1402-1418), both of which are incorporated by reference herein in their entirety. In relation to prostate cancer, PCWG3 criteria are preferred. Resistance against a certain therapy or drug can be determined e.g. from disease progression. Consequently, resistance is directly associated with reduced progression-free survival and a reduced time to no longer clinically benefiting (see PCWG3) from said therapy or drug.

Comparative terms, such as in "reduced chance to benefit", "reduced progression-free survival", "increased risk", etcetera, indicate a comparison to a control, such as a control sample or a control subject. Suitable controls are well-known to the skilled person in the field. Controls are generally elements with similar characteristics except for the feature at issue. For example, if the presence of an AR splice variant indicates that a tumor will have a reduced chance to benefit from ADT, it is meant that the tumor will have a reduced chance compared to a tumor that lacks said AR splice variant. Similarly, if the presence of an AR splice variant indicates that a patient will have a reduced progression-free survival, this means that the patient will have a reduced progression-free survival in comparison to a patient without the AR splice variant. As is well-known to the skilled person, if the method of the invention specifies a comparison to a control, this does not indicate that a control sample/subject should be analyzed for the presence of an AR splice variant each time that a sample is analyzed. Rather, values may be obtained from a single control sample (or mean or average values from multiple control samples) and said values may be used for the comparison in the method, even if said method is executed multiple times on different tumor samples.

As described herein before, in a first aspect, the present invention provides a method for determining if a tumor will be resistant to androgen deprivation therapy (ADT), the method comprising determining a) the presence or absence of an androgen receptor splice variant consisting of AR-V5 in a circulating tumor cell (CTC) enriched fluid sample of said tumor; and b) detecting Circulating Tumor Cells in said sample wherein the presence of the AR-V5 splice variant and CTC indicates that the tumor will be resistant to androgen deprivation therapy.

In a particular embodiments not part of the invention, the AR splice variant as described herein is AR-V1, AR-V2, AR-V3, AR-V9. AR-V10 or AR-V45. Preferably, the AR splice variant is selected from the group consisting of AR-V1, AR-V2, and AR-V10. In a further embodiment not part of the invention, the method comprises determining at least two, in particular at least three, AR splice variants; wherein the detection of at least one (in particular at least two, more in particular at least three) of said AR splice variants provides the indications disclosed herein.

In a further embodiment not part of the invention, the method comprises the detection of AR-V7 and at least one splice variant selected from AR-V1, AR-V2, AR-V3, AR-V5, AR-V9, AR-V10 and AR45; wherein the presence of AR-V1, AR-V2, AR-V3, AR-V5, AR-V7, AR-V9, AR-V10 or AR45 indicates that the tumor will be resistant to ADT. In another embodiment not part of the invention, the method comprises the detection of a) at least one AR splice variant selected from the group consisting of AR-V1, AR-V2, AR-V5 and AR-V10, and b) at least one AR splice variant selected from the group consisting of AR-V3, AR-V7 and AR-V9; wherein the presence of at least one AR splice variant from a) or b) indicates that the tumor will be non-responsive to ADT.

In another embodiment not part of the invention the method comprises the detection of AR-V5 and at least one splice variant selected from AR-V3, AR-V7, AR-V10 and AR-V45; wherein the presence of AR-V5 and at least one of AR-V3, AR-V7, AR-V10 and AR-V45 indicates that the tumor will be resistant to ADT. In another embodiment not part of the invention method comprises the detection of a) at least one AR splice variant selected from the group consisting of AR-V5 and AR-V10, and b) at least one AR splice variant selected from the group consisting of AR-V3, AR-V7 and AR-V45; wherein the presence of at least one AR splice variant from a) or b) indicates that the tumor will be non-responsive to ADT. In another embodiment not part of the invention, the method comprises the detection of at least one of AR-V3, AR-V5, AR-V7 and AR-V45; more in particular the detection of AR-V5; wherein the presence of AR-V3, AR-V5, AR-V7 or AR-V45; more in particular the presence of AR-V5 indicates that the tumor will be non-responsive to ADT. In another embodiment not part of the invention, the method comprises the detection of AR-V3, AR-V5, AR-V7 and AR-V45; wherein the presence of AR-V3, AR-V5, AR-V7 and AR-V45; in particular the presence of AR-V3, AR-V5, and AR-V7; more in particular the presence of AR-V5 indicates that the tumor will be non-responsive to ADT.

The present invention comprises the detection of AR-V5 and CTC count in a tumor sample of the patient; wherein the presence of AR-V5, and CTC indicates that the tumor will be non-responsive to ADT.

Both the circulating tumor cell (CTC) count (de Bono JS, Scher HI, Montgomery RB, et al. Circulating Tumor Cells Predict Survival Benefit from Treatment in Metastatic Castration-Resistant Prostate Cancer. Clinical Cancer Research 2008; 14: 6302-9; Scher HI, Heller G, Molina A, et al. Circulating Tumor Cell Biomarker Panel As an Individual-Level Surrogate for Survival in Metastatic Castration-Resistant Prostate Cancer. Journal of Clinical Oncology 2015; 33: 1348-55; Lorente D, Olmos D, Mateo J, et al. Decline in Circulating Tumor Cell Count and Treatment Outcome in Advanced Prostate Cancer. European Urology 2016; 70: 985-92) and prostate-specific antigen (PSA) level (Facchini, G., Caffo, O., Ortega, C., D'Aniello, C., Di Napoli, M., Cecere, S. C., et al. (2016). Very Early PSA Response to Abiraterone in mCRPC Patients: A Novel Prognostic Factor Predicting Overall Survival. Frontiers in Pharmacology, 7, 549-8; Rescigno, P., Lorente, D., Bianchini, D., Ferraldeschi, R., Kolinsky, M. P., Sideris, S., et al. (2016). Prostate-specific Antigen Decline After 4 Weeks of Treatment with Abiraterone Acetate and Overall Survival in Patients with Metastatic Castration-resistant Prostate Cancer. European Urology, 70(5), 724-731; Xu, X. S., Ryan, C. J., Stuyckens, K., Smith, M. R., Saad, F., Griffin, T. W., et al. (2015). Correlation between Prostate-Specific Antigen Kinetics and Overall Survival in Abiraterone Acetate-Treated Castration-Resistant Prostate Cancer Patients. Clinical Cancer Research, 21(14), 3170-3177) have previously been associated as prognostic markers in patients. However, and as evident from the instant application, fail to identify all patients that will not benefit from androgen deprivation therapy (ADT). Only when used with the determination of the androgen receptor splice varianst as disclosed herein, a conclusive differentiation of ADT responders over ADT non-responders can be reached.

Androgen deprivation therapy is known to the skilled person. It reduces androgen hormones, either with drugs or surgery. ADT is used most frequently for the treatment of prostate cancer. Surgical ADT comprises surgical castration. Preferably, ADT as used herein refers to a drug-based therapy, such as chemical castration or antiandrogen therapy. In a particular embodiment, ADT comprises administration of an antiandrogen. Particular antiandrogens for the methods described include cyproterone acetate, flutamide, nilutamide, bicalutamide, enzalutamide, abiraterone (also described herein as abiraterone acetate), apalutamide, EPI-001, ARN-509, and galeterone. In a more particular embodiment, said antiandrogen is an androgen synthesis inhibitor, such as abiraterone.

In yet another embodiment, the present invention provides a method for monitoring androgen deprivation therapy in a patient according to the method of the invention.

The subject or patient as described herein is preferably a mammal, in particular a human.

As described herein before, the present invention provides a computing system for predicting whether a tumor will be resistant to ADT.

The computing system may be cloud-based, wherein the storage medium is located on a remote server and wherein the computer program product may be executed using a remotely located processing engine.

### Examples

### Patient accrual

Men with progressive mCRPC (metastatic castration-resistant prostate cancer) were prospectively enrolled as defined by PCWG3 and RECIST1.1 guidelines (i.e. consecutive increases in serum PSA levels or radiographic evidence of disease progression in soft tissue or bone). Patients entering the study were starting a new standard-of-care treatment with abiraterone acetate or enzalutamide. A prior treatment with docetaxel or other chemotherapeutica was allowed, as also for other AR-targeted therapies (cfr. abiraterone before enzalutamide). The ethical review board of Antwerp University Hospital approved the study (Belgian registration number B300201524217), which was conducted according to the Declaration of Helsinki and the Good Clinical Practice guidelines of the International Conference on Harmonization. All patients provided written informed consent to participate in the study.

### Study design

This was a multicentric, prospective clinical study evaluating the predictive properties of a pretreatment AR splice variant (ARV) profile in circulating tumor cells from men with mCRPC, prior to starting a new hormonal therapy with abiraterone acetate or enzalutamide, according to stand-of-care practice. The study was conducted at 9 sites in 2 countries. Apart from GZA Sint-Augustinus (Antwerpen, BE) this includes AZ Sint-Jan Brugge - Oostende AV (Brugge, BE), AZ-Nikolaas (Sint-Niklaas, BE), AZ-KLINA (Brasschaat, BE), University Hospital Antwerp (Antwerpen, BE), University Hospital Gent (Gent, BE) and the non-Flemish actor Erasmus MC Cancer Institute (Rotterdam, NL). Peripheral blood samples were collected at baseline, at the time of therapy response assessment (9-12 weeks after start) and a final blood draw at disease progression (clinical, biochemical and radiological), according to PCWG3 and RECIST1.1 criteria. Peripheral blood samples were collected in CellSave (Janssen Diagnostics) and EDTA (Becton Dickinson) tubes, for enumeration and ARV expression profiling, respectively. Acquired samples from the different participating hospitals were processed and stored within 8-10 hours after collection. All samples were prepared, processed and analysed in the central laboratory of GZA Sint-Augustinus, thereby eliminating technical variability between hospitals. Sample transport from participating Belgian and Dutch hospitals towards the central laboratory was performed by a courier service, taking care that the samples were processed within 12 hours of collection. PSA measurements were performed every month, and radiological therapy response assessments were performed between 2-4 months. During the patient accrual phase the clinical investigators were blinded for the AR splice variant status of the patients.

### CTC enrichment from whole blood using CellSearch®

For CTC enumeration of patient samples, 7.5 mL whole blood samples was collected in CellSave Vacutainer tubes (Janssen Diagnostics) and processed within 72 hours after collection. Standard protocols and reagents for CTC enrichment with the FDA-approved CellSearch® CTC kit (Janssen Diagnostics LLC, Raritan, NJ, USA) were applied. For profiling studies, 7.5 mL EDTA whole blood samples was collected and processed within 8 hours. Standard protocols and reagents for CTC isolation with the CellSearch® Profile kit (Janssen Diagnostics LLC, Raritan, NJ, USA) were applied.

### RNA isolation, cDNA synthesis and preamplification.

Upon enrichment and lysis of CTCs, the RNA was isolated with the AllPrep DNA/RNA Micro Kit (Qiagen) according to protocol described by Sieuwerts et al. (Breast Cancer Res Treat, 2008) and stored at -80°C. Complementary DNA (cDNA) synthesis and pre-amplification was performed on 50% of the isolated RNA with the RevertAid™ H Minus First Strand cDNA Synthesis Kit (Fermentas, St. Leon-Rot, Germany) and the TaqMan™ PreAmp amplification method (Applied Biosystems), respectively. Used primer/probe sequences for targeted amplification or AR and androgen receptor splice variants are presented in table1. The cDNA synthesis existed of 4 steps. In the first step oligo (dT) and random hexamer primers are added, that hybridize to the poly(A) tail or randomly along the mRNA, respectively, resulting in the generation of full-length cDNA from poly(A) tailed mRNA. The random hexamer primer is a mixture of single-stranded random hexanucleotides with 5'- and 3'-hydroxyl ends. The samples are incubated for 5 minutes at 70°C and are cooled down to 0°C which allows for the denaturation of the RNA and annealing of primers. A mix of dNTPs (dATP, dGTP, dCTP and dTTP), reaction buffer and Ribolock RNase-inhibitor is added to the sample. The samples are heated at 25°C during 5 minutes. The reaction buffer is a mixture of 250 mM Tris-HCI (pH 8.3), 250 mM KCI, 20 mM MgCl₂, 50 mM DTT, providing optimal conditions for reverse transcriptase activity. KCI neutralizes the charge off the backbone of DNA/RNA, which facilitates the annealing of primers to template by reducing the repulsion between the negatively charged DNA and RNA strands. MgCl₂ is a necessary cofactor for enzymatic activity. DTT or dithiothreitol is reducing agent for disulfide bonds and stabilizes enzymes and proteins (containing sulfhydryl groups). Ribolock RNase-inhibitor protects RNA from degradation at temperatures up to 55°C, it inhibits the activity of RNases A, B and C by binding them in a noncompetitive mode at a 1:1 ratio. Reverse transcriptase is an RNA-directed DNA polymerase, which synthesizes complementary DNA (cDNA) strand from a RNA template. The kit uses RevertAid™ H Minus M-MuLV Reverse Transcriptase, which has a point mutation that completely eliminates RNase H activity. In a -RT sample no reverse transcriptase is added to the reaction mixture, but replaced by TE-buffer. TE-buffer is a mixture of EDTA and Tris-HCL, ETDA chelates cations and Tris-HCI is a buffer. The final step is adding RNase H to the samples, which degrades the RNA. cDNA synthesis was performed using the RevertAid H Minus First Strand cDNA Synthesis Kit and Applied Biosystems® GeneAmp® PCR System 9700 (Life technologies).

Because of the low input of RNA pre-amplification of target molecules is preferred. During this progress the quantity of specific cDNA targets is increased using TaqMan® PreAmp Master Mix. The primers of the target genes, the control genes for qRT-PCR (i.e. EpCAM and PTPRC), and the housekeeping genes for qRT-PCR (i.e. GUSB, HMBS, HPRT1) are pooled. The developed TaqMan qRT-PCR assays for the AR-V target genes are presented in Table 1. This pool of primers is mixed with the pre-amplification master mix and added to the sample. First, DNA polymerase synthesizes the complement strand of cDNA. In the following cycli DNA polymerase replicates both strands. The TaqMan® PreAmp Master Mix was used with a GeneAmp® PCR System 9700 (Life technologies). Upon preamplification, samples were diluted 5-10 times with TE-buffer.

**Table 1. Used primer and probe sequences for targeted pre-amplification and detection of AR and androgen receptor splice variants**

| **ARV** | **Forward primer** | **Reverse primer** | **Probe sequence** |
|---|---|---|---|
| AR-V1 | GTCGTCTTCGGAAATGTTA (SEQ ID NO: 27) | TGAGAGTCTGAAGGTAGTCT (SEQ ID NO: 28) | ACTCTGGGAGCAGCTGTTGTT (SEQ ID NO: 29) |
| AR-V2 | CTGGGAGGGAAACAGAAG (SEQ ID NO: 30) | AGAAACAACAACAGCTGCT (SEQ ID NO: 31) | TGTGCGCCAGCAGAAATGATT (SEQ ID NO: 32) |
| AR-V3 | CTTCTGGGTGTCACTATG (SEQ ID NO: 33) | CTTGGGGTTAGTGTCTGAT (SEQ ID NO: 34) | AGAGCCGCTGAAGGATTTTTCAGA (SEQ ID NO: 35) |
| AR-V4 | TCTTGTCGTCTTCGGAAAT (SEQ ID NO: 36) | CTTGGGGTTAGTGTCTGAT (SEQ ID NO: 37) | TGACTCTGGGAGGATTTTTCAGAA (SEQ ID NO: 38) |
| AR-V5 | CTTGTCGTCTTCGGAAAT (SEQ ID NO: 39) | CAAAGAATTGTGGGTAGGAAGCAG (SEQ ID NO: 40) | AGGGATGACTCTGGGAGACTAGAA (SEQ ID NO: 41) |
| AR-V6 | GGATGACTCTGGGAGCA (SEQ ID NO: 42) | CCTGAGGGTCTTTGGAATT (SEQ ID NO: 43) | AACGTGGAACTCAGTTACTGGGAT (SEQ ID NO: 44) |
| AR-V7 | GTCCATCTTGTCGTCTTC (SEQ ID NO: 45) | GCAAGTCAGCCTTTCTTCA (SEQ ID NO: 46) | GGGAGAAAAATTCCGGGTTGGC (SEQ ID NO: 47) |
| AR-V8 | CGAAATACCCGAAGAAAGA (SEQ ID NO: 48) | GGACAA TTTTTCCTTCGGA (SEQ ID NO: 49) | TGTTCTCCCAGGGAAACAGAAGT (SEQ ID NO: 50) |
| AR-V9 | GTCTTCGGAAATGTTATGAA (SEQ ID NO: 51) | CTGCGTGTTTTTCCCTTAG (SEQ ID NO: 52) | TCTGGGAGACAACTTACCTGAGC (SEQ ID NO: 53) |
| AR-V10 | TTGTCCATCTTGTCGTCT (SEQ ID NO: 54) | TCATGATACTCACAACTAC (SEQ ID NO: 55) | TACCTCACAGGGATGTTGTG (SEQ ID NO: 56) |
| AR-V12 | AGAGAGACAGCTTGTACA (SEQ ID NO: 57) | CCATCATTTCCGGAAAGT (SEQ ID NO: 58) | CTTGCCTGATTGCGAGAGAGCT (SEQ ID NO: 59) |
| AR-V13 | GGAATTCCTGTGCATGAA (SEQ ID NO: 60) | GCAACTACTAGAGAAAAGGA (SEQ ID NO: 61) | CAGCATTAGATTATGGGTCCTTCAC (SEQ ID NO: 62) |
| AR-V14 | CATCCTGCTCAAGACGCTTCTACCA (SEQ ID NO: 63) | GCAACTACTAGAGAAAAGGA (SEQ ID NO: 64) | GTGCAGCCTGATTATGGGTC (SEQ ID NO: 65) |
| AR-fl | CTGCTCAAGACGCTTCTA (SEQ ID NO: 66) | ATCATTTCCGGAAAGTCCA (SEQ ID NO: 67) | TCCGTGCAGCCTATTGCGAG (SEQ ID NO: 68) |
| AR45 | CTGTCACTTTTTCCCATGA (SEQ ID NO: 69) | AAGCTTCATCTCCACAGA (SEQ ID NO: 70) | TGGCTTCACAGTTTGGAGACTG (SEC ID NO: 71) |

### qRT-PCR for detection of androgen receptor splice variants

Quantitative reverse-transcriptase polymerase chain reaction (qRT-PCR) assays were used to quantitate the presence of different mRNA transcripts of AR in enriched CTC fractions. Pre-amplified cDNA products were diluted and subjected to real-time PCR amplification for AR and ARV transcripts (see table 1), and 3 reference genes (*GUSB, HMBS* and *HPRT1*) to control for sample loading and RNA integrity. To assess epithelial signal towards WBC background, the epithelial specific markers *EPCAM* and *CK19* and the white blood cell marker *PTPRC,* which encodes for CD45, was included. Fluorigenic PCR reactions were performed in 40 cycles in a final volume of 20 µL using custom-made TaqMan™ Gene Expression Assays for the AR isoforms and Taqman™ Universal PCR Master Mix No AmpErase UNG on a 7900HT Fast Real-time PCR System (all Applied Biosystems). Each PCR run was accompanied by a calibrator sample, from which the mean Ct values were used to assess the inter-run reproducibility. Negative controls included a buffer and minus RT sample. Only samples with a Ct value of <35 for each of the individual 3 reference genes, and a median Ct value for all reference genes of <30, were considered of sufficient quality and quantity to be included in the analyses (as described by Onstenk et al, 2015, Eur Urol.) PCR quantification was performed using the Pfaffl and Livak method with normalization to the reference genes and calibrator sample.

### AR and ARV Multiplex Amplification of Specific Targets for Resequencing and sequence analysis

Pre-amplified cDNA products were diluted and subjected to a multilplex PCR for amplification, sequence adaptor ligation and indexing of AR and ARV transcripts from a given patient sample. Hereto diluted preamplified samples were mixed with the ARV MASTR mastermix and subjected to 20 cycli of PCR with a annealing temperature of 60°C. Upon MASTR amplification, PCR amplicons were verified and subjected to quality control using a Genescan (Applied Biosystems) measurement. Upon library quantification and pooling, PCR amplicon pools were subjected to sequencing by synthesis using a MiSeq (Illumina) with v2 chemistry (2x251 cycli). Upon sequencing, demultiplexed fastq files were exported from the MiSeq machine and subjected to trimming using Skewer software with a sequence length cutoff of 80 nucleotides. Trimmed fastq files were either subjected to a supervised FASTA string search with 0% mismatch or were subjected to alligning with the Burrow Wheeler Algorithm (BWA-mem) using an indexed ARV reference transcript file of known ARV. Upon BWA mapping, SAMTOOLS was used to infer the raw counts per ARV, which were correlated to counted hits from the targeted FASTA string search with 0% mismatch. Raw counts were normalized to total ARV sequence coverage, resulting in a normalized expression value of a number of ARV transcripts/1000 reads.

### Clinical outcome and statistical analysis

For the abiraterone- and enzalutamide-treated patients, the proportion of patients with a PSA response of ≥30% or ≥50% from baseline PSA levels was determined. Additionally, the clinical progression-free survival or the time between start of therapy and time to no longer clinically benefitting was determined. Assessment of biochemical and clinical/radiographic progression was performed, according to the PCWG2 and RECIST1.1 guidelines. Overall survival was defined as the time to death from any cause. In abiraterone and enzalutamide-treated patients, the clinical outcomes to the ARV profile and/or AR isoform status (positive vs negative for a particular ARV) were examined and compared. Differences in PSA response rates were assessed using a Chi-Square/Fisher's exact test. Based on these results, a predictive model was generated, which was able to discriminate the patients with short and long PFS with highest sensitivity and specificity. Model construction was performed using neural networks, random forests and support vector machine algorithms and validated using the leave-one-out cross-validation test. Only ARVs with P<0.001 were kept to generate a ARV signature discriminating responder and non-responder groups. Kaplan-Meier analysis was used to define outcomes in PFS and OS. Survival differences were determined using the log-rank test. Uni- and multi-variable Cox regression analysis was performed to assess the effect of the ARV presence on the time- to-event outcomes. All tests were performed in R, with a two-sided P value <0,05 as being considered as statistically significant.

### Results

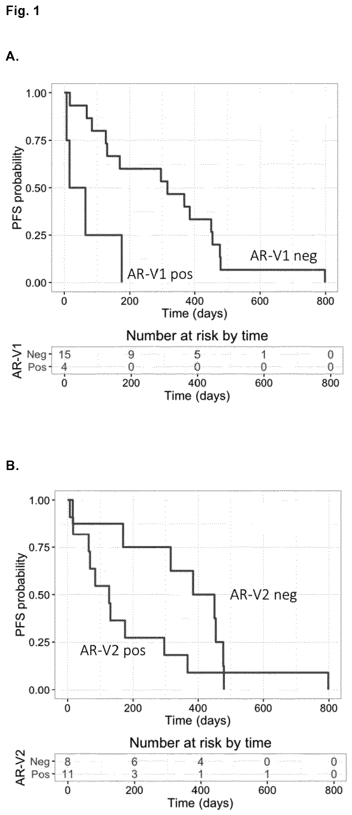

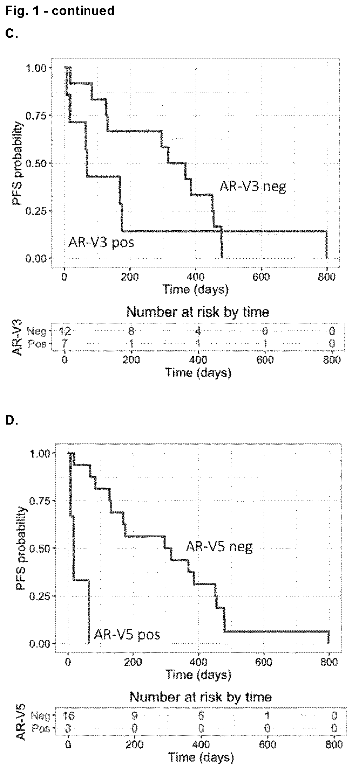

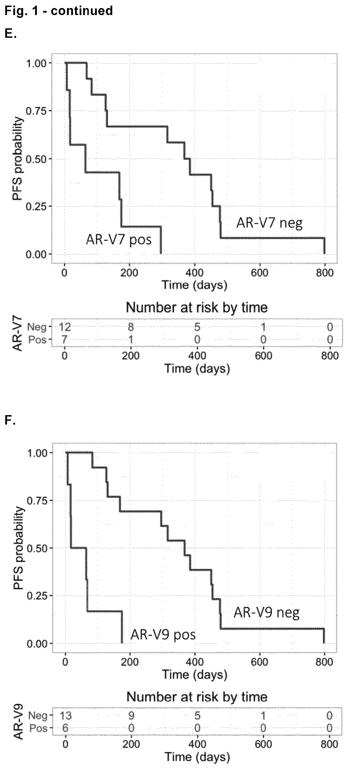

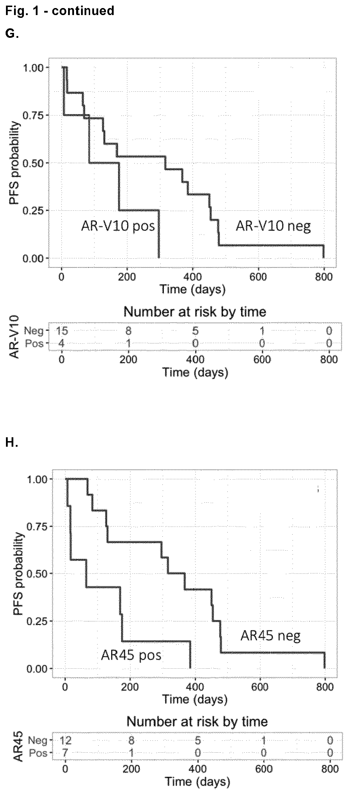

Results from Kaplan-Meier (KM) analysis on progression-free survival (PFS) and AR-V status at baseline (i.e. at start of new line of systemic therapy with abiraterone and/or enzalutamide) are presented in Fig. 1 A-H. These KM plots show a clear association between the presence of a particular AR splice variants and treatment resistance, as measured by a shorter or decreased PFS. After performing a log-rank test statistical significance was obtained for AR-V1 (p=0.0033), AR-V5 (p<0.0001), AR-V7 (p=0.0025), AR-V9 (p<0.0001), and AR45 (p=0.013).

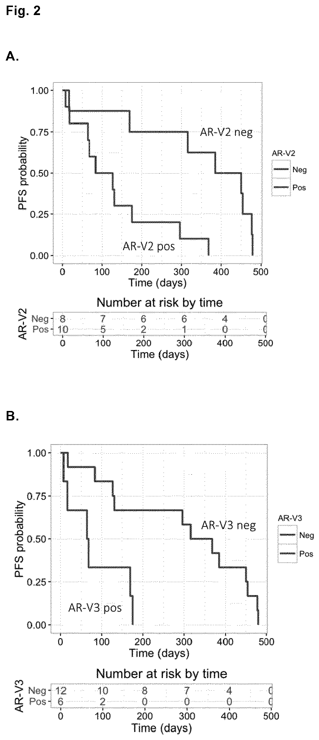

While not reaching statistical significance in this particular experiment, a clear trend towards significance was observed for AR-V2, AR-V3 and AR-V10. For example, a single AR-V2 and - V3 positive patient with a long PFS distorted the analysis, as depicted in Figure 1B and 1C, respectively. Removal of this outlier patient led to a significant correlation between AR-V2 and AR-V3 status and PFS (p=0.0038 and p=0.0029, respectively), as presented in Figure 2A en 2B.

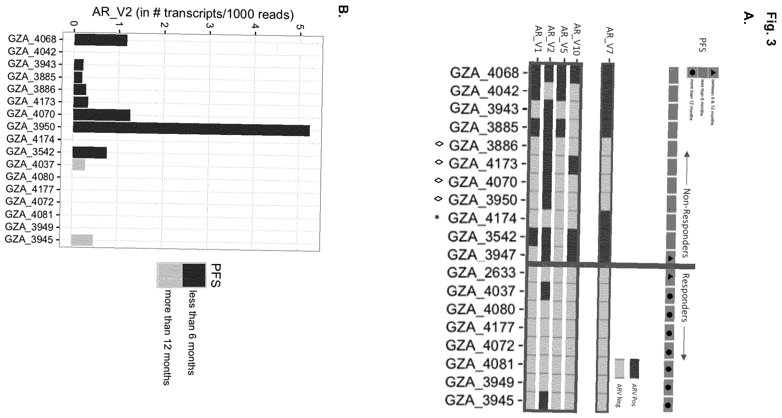

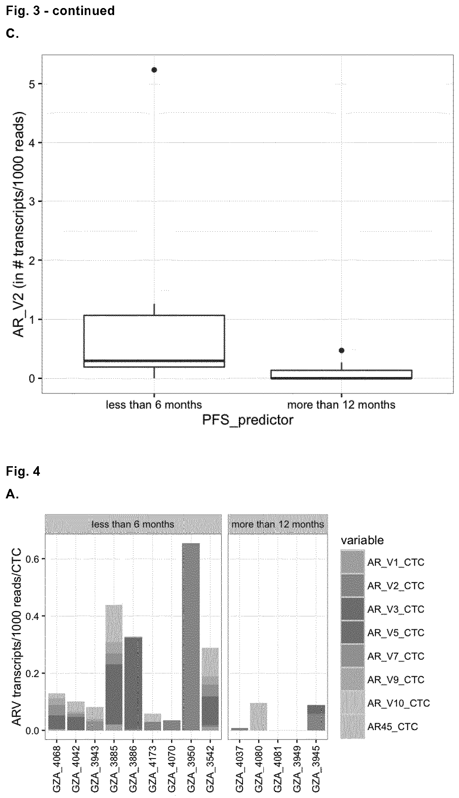

Next, the ability of the claimed splice variants, i.e. selected from the group consisting of AR-V1, AR-V2, AR-V5 and AR-V10 were checked for their predictive power to discriminate responders from non-responders while on abiraterone or enzalutamide treatment, as measured by the time to clinical progression. Additionally, the discriminatory power of the selected set of ARVs were benchmarked against the current potential predictor in the field of abiraterone/enzalutamide resistance, namely AR-V7. As presented in Figure 3A, AR-V7 detection in enriched CTC fractions at baseline was capable of identifying 7/11 (63.6%) of the patients who demonstrated clinical progression within 6 months. Four patients (marked by 0) would have been missed when using a negative predictor solely on the basis of AR-V7 positivity. When measuring the presence of AR-V1, AR-V2, AR-V5 and AR-V10 in the baseline sample, 10/11 (90.9%) of the patients with short PFS could be identified. Using this set of claimed ARVs one patient with short PFS (marked by *) would have been missed, who however was identified by the AR-V7 assay. Additionally, it could be noted how two patients with a PFS > 12 months were positive for AR-V2, with overall demonstrating a trend of lower AR-V2 transcript levels, as presented in Figure 3B and 3C.

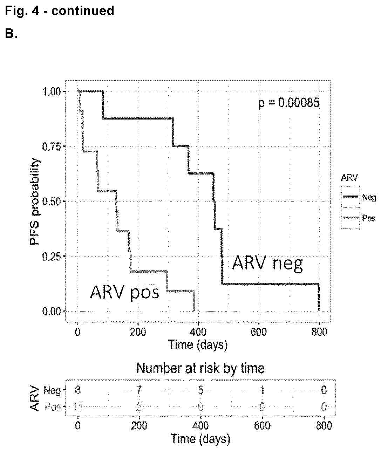

Finally it could be noted how AR splice variants as such are more abundantly present in patients demonstrating a short progression free survival, as presented by the 'less than 6 months' group in Figure 4A. In general, a heterogeneous ARV expression pattern can be observed between and within patients. In general one can conclude that patients demonstrating a long PFS (as presented by the 'more than 12 months' group in Figure 4A) have lower and/or fewer AR splice variants. Additionally it was noted how the presence of an ARV (regardless which ARV) is correlated with a shorter PFS (p < 0.00085), as presented in figure 4B.

### ARV Analysis in a representative Patient Cohort

Men with progressive mCRPC (metastatic castration-resistant prostate cancer) were prospectively enrolled as defined by PCWG3 and RECIST1.1 guidelines (i.e. consecutive increases in serum PSA levels or radiographic evidence of disease progression in soft tissue or bone). Patients entering the study were starting a new standard-of-care treatment with abiraterone acetate or enzalutamide. A prior treatment with docetaxel or other chemotherapeutica was allowed, as also for other AR-targeted therapies (cfr. abiraterone before enzalutamide).

### Cohort profile

149 baseline samples were available with the following profile;
Chemotherapy Status
   - Naïve:92
   - Pretreated:57
Age at start abi/enza
   - Median(IQR):75.5y(69y-80y)
Patient markers
   - CTC : <5:87, ≥5:58, Undetermined:4
   - PSA : Median(IQR):25.20(11.71-91.82)
   - LDH : Median(IQR):314(212-608)
   - AP : Median (IQR): 98.5 (71.25 - 153)

### Blood collection and processing

Blood collection and processing for circulating tumor cell (CTC) enumeration (within 72 hours) or CTC enrichment for transcriptional analysis (within 8 hours) were performed using the FDA-cleared CellSearch CTC and Profile technology, respectively. Profile-enriched CTC fractions were lysed with 250µL RNeasy RLT+ buffer (Qiagen BV, The Netherlands) and stored at - 80°C until RNA isolation.

### RNA isolation, cDNA synthesis and AR-V preamplification from enriched CTC fractions

RNA was isolated from CTC lysates with the AllPrep DNA/RNA Micro Kit (Qiagen). Complementary DNA (cDNA) synthesis and AR-V pre-amplification was performed on 25-50% of the isolated RNA, using the RevertAidTM H Minus First Strand cDNA and TaqManTM PreAmp amplification kit, respectively, in a GeneAmp® PCR System 9700 (Life technologies).

### Full-length AR and AR splice variant (ARV) Multiplex Amplification of Specific Targets for Resequencing and qRT-PCR validation

Pre-amplified cDNA was subjected to a 20-cycle multiplex PCR for amplification, sequence adaptor ligation and indexing of full-length AR and AR splice variant (ARV) transcripts using Multiplex Amplification of Specific Targets for Resequencing (MASTR) technology (Multiplicom NV, Belgium). Amplicon libraries were verified by fragment analysis using Genescan (Applied Biosystems). Pooled libraries were sequenced on a MiSeq (Illumina) with v2 chemistry (2x251 cycli).

### ARV-Seq Sequence analysis

Demultiplexed fastq files were trimmed using Skewer software version 0.1.117 with a sequence length cutoff of 80 nucleotides. Filtered reads were aligned with the Burrow Wheeler Algorithm (BWA-mem) using an indexed ARV reference transcript FASTA, encompassing exon and cryptic exon (CE) signature sequences. SAMTOOLS was used to infer the read counts per ARV. Raw read counts were subsequently normalized and expressed as the number of ARV transcripts/1000 reads.

### Results

Of the 149 baseline samples, 133 could be analysed, 16 failed (sequencing quality). The Androgen receptor splice variants only represent a small fraction of the total reads (mean 1,45% median 0,03%). As evident from the below table, AR-V3 is the most prevalent malignant AR-V (confirms the earlier results) together with AR-V45. AR-V1 and AR-V2 are present in practically all patients.

**Table 2. AR-V isoforms prevalence in mCRPC**

| AR-V | Neg | Pos | Prevalence in mCRPC |
|---|---|---|---|
| AR45 | 86 | 47 | 35,3% |
| AR-V1 | 32 | 101 | 75,9% |
| AR-V2 | 34 | 99 | 74,4% |
| AR-V3 | 81 | 52 | 39,1% |
| AR-V5 | 116 | 17 | 12,8% |
| AR-V7 | 101 | 32 | 24,1% |
| AR-V9 | 114 | 19 | 14,3% |

Based on uni-variate and multi-variate analysis it was further assessed how these AR-Vs are associated with Progression Free Survival. Per reference to the below table, CTCs, AR-45, AR-V3, AR-V5 and AR-V7 are associated in univariate, but in case of multivariate analysis only AR-V5 and CTC remain. These results underscore AR-V5 as a prognostic marker allowing to differentiate ADT responders over ADT non-responders when taken in Isolation, in particular when combined with CTC.

**Table 3. AR-V isoforms and survival**

| | | **Univariate** | | | | **Multivariate** | | |
|---|---|---|---|---|---|---|---|---|
| **Variable** | **categories** | **p (Logrank)** | **HR** | **95%CI** | **p (Cox PH)** | **HR** | **95%CI** | **p (Cox PH)** |
| AR45 | pos/neg | **0.025** | 1.701 | 1.064 - 2.72 | **0.0265** | 1.153 | 0.6097 - 2.179 | 0.66191 |
| AR-V1 | pos/neg | 0.47 | 0.82 | 0.4787 - 1.405 | 0.47 | - | - | - |
| AR-V2 | pos/neg | 0.44 | 0.8126 | 0.4818 -1.37 | 0.436 | - | - | - |
| AR-V3 | pos/neg | **0.026** | 1.679 | 1.058 - 2.664 | **0.0278** | 1.012 | 0.4869 - 2.103 | 0.97451 |
| AR-V5 | pos/neg | **0.00029** | 2.853 | 1.578 - 5.159 | **0.000521** | 2.327 | 1.0959 - 4.940 | **0.02792** |
| AR-V7 | pos/neg | **0.0043** | 2.048 | 1.239 - 3.387 | **0.00519** | 1.136 | 0.5625 - 2.294 | 0.72223 |
| AR-V9 | pos/neg | 0.49 | 1.22 | 0.6886 - 2.162 | 0.495 | - | - | - |
| CTC | <5/≥5 | **0.00058** | 2.232 | 1.397 - 3.567 | **0.000789** | 2.078 | 1.2591 - 3.429 | **0.00422** |

## Claims

1. An in vitro method for determining if a tumor will be resistant to androgen deprivation therapy (ADT), the method comprising determining a) the presence or absence of an androgen receptor splice variant consisting of AR-V5 in a circulating tumor cell (CTC) enriched fluid sample of said tumor; and b) detecting Circulating Tumor Cells in said sample
wherein the presence of the AR-V5 splice variant and CTC indicates that the tumor will be resistant to androgen deprivation therapy.

2. The method of claim 1, wherein the androgen deprivation therapy comprises antiandrogen therapy.

3. The method of claim 2, wherein the antiandrogen therapy comprises administration of abiraterone acetate or enzalutamide.

4. The method of any one of the previous claims, wherein the tumor is a prostate tumor.

5. The method of any one of the previous claims, wherein the tumor is a castration-resistant prostate cancer (CRPC).

6. The method of any one of the previous claims, wherein the tumor fluid sample is a blood sample or a sample derived from a blood sample.

7. The method of any one of the previous claims, further comprising determining one or more additional parameters from the patient carrying the tumor, wherein the additional parameters are selected from the group consisting of prostate-specific antigen level and the expression level of one or more androgen receptor splice variants.

8. A computing system for predicting whether a tumor will be resistant to androgen deprivation therapy (ADT), the computing system comprising
- a storage medium comprising a database structure for accepting androgen receptor splice variant and tumor marker information from a sample of a tumor, wherein the androgen splice variant is AR-V5 and wherein the tumor marker is CTC; and
- a computer program product that, when executed on a processing engine,
a) determines the presence or absence of an AR-V5 androgen receptor splice variant in the sample based on the androgen receptor splice variant information in the database structure,
b) determines the presence or absence of the CTC tumor marker; and
c) indicates that the tumor will be resistant to androgen deprivation therapy if the presence of the androgen receptor splice variant has been determined in step a) and the presence of the CTC tumor marker has been determined in step b).

## Patentansprüche

1. In-vitro-Verfahren zum Ermitteln, ob ein Tumor gegen eine Androgenentzugstherapie (ADT) resistent ist, wobei das Verfahren a) das Ermitteln des Vorhandenseins oder der Abwesenheit einer Androgenrezeptor-Spleißvariante, bestehend aus AR-V5, in einer mit zirkulierenden Tumorzellen (CTC) angereicherten Flüssigkeitsprobe des Tumors umfasst; und b) das Nachweisen von zirkulierenden Tumorzellen in der Probe, wobei das Vorhandensein der AR-V5-Spleißvariante und der CTC angibt, dass der Tumor gegen die Androgenentzugstherapie resistent ist.

2. Verfahren nach Patentanspruch 1, wobei die Androgenentzugstherapie eine Antiandrogentherapie umfasst.

3. Verfahren nach Patentanspruch 2, wobei die Antiandrogentherapie die Verabreichung von Abirateronacetat oder Enzalutamid umfasst.

4. Verfahren nach einem der vorhergehenden Patentansprüche, wobei der Tumor ein Prostatatumor ist.

5. Verfahren nach einem der vorhergehenden Patentansprüche, wobei der Tumor ein kastrationsresistenter Prostatakrebs (CRPC) ist.

6. Verfahren nach einem der vorhergehenden Patentansprüche, wobei die Tumorflüssigkeitsprobe eine Blutprobe oder eine von einer Blutprobe abgeleitete Probe ist.

7. Verfahren nach einem der vorhergehenden Patentansprüche, ferner umfassend das Ermitteln eines oder mehrerer zusätzlicher Parameter von dem Patienten, der den Tumor trägt, wobei die zusätzlichen Parameter ausgewählt sind aus der Gruppe bestehend aus dem prostataspezifischen Antigen-Level und dem Expressionslevel von einer oder mehreren Androgenrezeptor-Spleißvarianten.

8. Computersystem zum Vorhersagen, ob ein Tumor gegenüber einer Androgenentzugstherapie (ADT) resistent ist, wobei das Computersystem ein Speichermedium umfasst, das eine Datenbankstruktur zum Aufnehmen von Androgenrezeptor-Spleißvariante- und Tumormarkerinformationen von einer Probe eines Tumors umfasst, wobei die Androgenspleißvariante AR-V5 ist, und wobei der Tumormarker CTC ist; und ein Computerprogrammprodukt, das, wenn es auf einer Verarbeitungs-Engine ausgeführt wird, das Vorhandensein oder die Abwesenheit einer AR-V5-Androgenrezeptor-Spleißvariante in der Probe basierend auf den Informationen über die Androgenrezeptor-Spleißvariante in der Datenbankstruktur ermittelt, das Vorhandensein oder die Abwesenheit des CTC-Tumormarkers ermittelt und angibt, dass der Tumor gegenüber einer Androgenentzugstherapie resistent ist, wenn das Vorhandensein der Androgenrezeptor-Spleißvariante in Schritt a) ermittelt wurde und das Vorhandensein des CTC-Tumormarkers in Schritt b) ermittelt wurde.

## Revendications

1. Méthode in vitro destinée à déterminer si une tumeur sera résistante au traitement par privation androgénique (TPA), la méthode consistant à a) déterminer la présence ou l'absence d'un variant d'épissage du récepteur des androgènes consistant en AR-V5 dans un échantillon de liquide enrichi en cellules tumorales circulantes (CTC) de ladite tumeur ; et b) détecter des cellules tumorales circulantes dans ledit échantillon, étant entendu que la présence du variant d'épissage AR-V5 et de CTC indique que la tumeur sera résistante au traitement par privation androgénique.

2. Méthode selon la revendication 1, dans laquelle le traitement par privation androgénique comprend le traitement anti-androgénique.

3. Méthode selon la revendication 2, dans laquelle le traitement anti-androgénique comprend l'administration d'acétate d'abiratérone ou d'enzalutamide.

4. Méthode selon l'une quelconque des revendications précédentes, dans laquelle la tumeur est une tumeur de la prostate.

5. Méthode selon l'une quelconque des revendications précédentes, dans laquelle la tumeur est un cancer de la prostate résistant à la castration (CPRC).

6. Méthode selon l'une quelconque des revendications précédentes, dans laquelle l'échantillon de liquide tumoral est un échantillon de sang ou un échantillon dérivé d'un échantillon de sang.

7. Méthode selon l'une quelconque des revendications précédentes, comprenant en outre la détermination d'un ou plusieurs paramètres supplémentaires du patient porteur de la tumeur, dans laquelle les paramètres supplémentaires sont sélectionnés dans le groupe constitué du taux d'antigène prostatique spécifique et du niveau d'expression d'un ou plusieurs variants d'épissage du récepteur des androgènes.

8. Système informatique permettant de prédire si une tumeur sera résistante au traitement par privation androgénique (TPA), ledit système contenant un support de stockage comprenant une structure de base de données destinée à accueillir les informations relatives au variant d'épissage du récepteur des androgènes et au marqueur tumoral dans un échantillon de tumeur, étant entendu que le variant d'épissage du récepteur des androgènes est AR-V5 et que le marqueur tumoral correspond aux CTC ; et un produit, c.-à-d. un programme informatique, qui, lorsqu'il est exécuté sur un moteur de traitement, détermine la présence ou l'absence d'un variant d'épissage du récepteur des androgènes AR-V5 dans l'échantillon en fonction des informations relatives au variant d'épissage du récepteur des androgènes dans la structure de la base de données, détermine la présence ou l'absence du marqueur tumoral CTC, et indique que la tumeur sera résistante au traitement par privation androgénique si la présence du variant d'épissage du récepteur des androgènes a été déterminée à l'étape a) et si la présence du marqueur tumoral CTC a été déterminée à l'étape b).


### SEQUENCE LISTING

<110> Sint Augustinus Universiteit Antwerpen
<120> Androgen receptor splice variants and androgen deprivation therapy
<130> GZA-001
<150> EP16172764.9
   <151> 2016-06-02
<160> 71
<170> BiSSAP 1.3.6
<210> 1
   <211> 920
   <212> PRT
   <213> Homo sapiens
<400> 1
<210> 2
   <211> 3569
   <212> DNA
   <213> Homo sapiens
<400> 2
   taataactca gttcttattt gcacctactt cagtggacac tgaatttgga aggtggagga      60
   ttttgttttt ttcttttaag atctgggcat cttttgaatc tacccttcaa gtattaagag     120
   acagactgtg agcctagcag ggcagatctt gtccaccgtg tgtcttcttc tgcacgagac     180
   tttgaggctg tcagagcgct ttttgcgtgg ttgctcccgc aagtttcctt ctctggagct      240
   tcccgcaggt gggcagctag ctgcagcgac taccgcatca tcacagcctg ttgaactctt     300
   ctgagcaaga gaaggggagg cggggtaagg gaagtaggtg gaagattcag ccaagctcaa     360
   ggatggaagt gcagttaggg ctgggaaggg tctaccctcg gccgccgtcc aagacctacc     420
   gaggagcttt ccagaatctg ttccagagcg tgcgcgaagt gatccagaac ccgggcccca     480
   ggcacccaga ggccgcgagc gcagcacctc ccggcgccag tttgctgctg ctgcagcagc     540
   agcagcagca gcagcagcag cagcagcagc agcagcagca gcagcagcag cagcaagaga     600
   ctagccccag gcagcagcag cagcagcagg gtgaggatgg ttctccccaa gcccatcgta     660
   gaggccccac aggctacctg gtcctggatg aggaacagca accttcacag ccgcagtcgg     720
   ccctggagtg ccaccccgag agaggttgcg tcccagagcc tggagccgcc gtggccgcca     780
   gcaaggggct gccgcagcag ctgccagcac ctccggacga ggatgactca gctgccccat     840
   ccacgttgtc cctgctgggc cccactttcc ccggcttaag cagctgctcc gctgacctta    900
   aagacatcct gagcgaggcc agcaccatgc aactccttca gcaacagcag caggaagcag     960
   tatccgaagg cagcagcagc gggagagcga gggaggcctc gggggctccc acttcctcca    1020
   aggacaatta cttagggggc acttcgacca tttctgacaa cgccaaggag ttgtgtaagg    1080
   cagtgtcggt gtccatgggc ctgggtgtgg aggcgttgga gcatctgagt ccaggggaac    1140
   agcttcgggg ggattgcatg tacgccccac ttttgggagt tccacccgct gtgcgtccca    1200
   ctccttgtgc cccattggcc gaatgcaaag gttctctgct agacgacagc gcaggcaaga    1260
   gcactgaaga tactgctgag tattcccctt tcaagggagg ttacaccaaa gggctagaag 1320
   gcgagagcct aggctgctct ggcagcgctg cagcagggag ctccgggaca cttgaactgc 1380
   cgtctaccct gtctctctac aagtccggag cactggacga ggcagctgcg taccagagtc 1440
   gcgactacta caactttcca ctggctctgg ccggaccgcc gccccctccg ccgcctcccc 1500
   atccccacgc tcgcatcaag ctggagaacc cgctggacta cggcagcgcc tgggcggctg 1560
   cggcggcgca gtgccgctat ggggacctgg cgagcctgca tggcgcgggt gcagcgggac 1620
   ccggttctgg gtcaccctca gccgccgctt cctcatcctg gcacactctc ttcacagccg 1680
   aagaaggcca gttgtatgga ccgtgtggtg gtggtggggg tggtggcggc ggcggcggcg 1740
   gcggcggcgg cggcggcggc ggcggcggcg gcggcggcga ggcgggagct gtagccccct 1800
   acggctacac tcggccccct caggggctgg cgggccagga aagcgacttc accgcacctg 1860
   atgtgtggta ccctggcggc atggtgagca gagtgcccta tcccagtccc acttgtgtca 1920
   aaagcgaaat gggcccctgg atggatagct actccggacc ttacggggac atgcgtttgg 1980
   agactgccag ggaccatgtt ttgcccattg actattactt tccaccccag aagacctgcc 2040
   tgatctgtgg agatgaagct tctgggtgtc actatggagc tctcacatgt ggaagctgca 2100
   aggtcttctt caaaagagcc gctgaaggga aacagaagta cctgtgcgcc agcagaaatg 2160
   attgcactat tgataaattc cgaaggaaaa attgtccatc ttgtcgtctt cggaaatgtt 2220
   atgaagcagg gatgactctg ggagcccgga agctgaagaa acttggtaat ctgaaactac 2280
   aggaggaagg agaggcttcc agcaccacca gccccactga ggagacaacc cagaagctga 2340
   cagtgtcaca cattgaaggc tatgaatgtc agcccatctt tctgaatgtc ctggaagcca 2400
   ttgagccagg tgtagtgtgt gctggacacg acaacaacca gcccgactcc tttgcagcct 2460
   tgctctctag cctcaatgaa ctgggagaga gacagcttgt acacgtggtc aagtgggcca 2520
   aggccttgcc tggcttccgc aacttacacg tggacgacca gatggctgtc attcagtact 2580
   cctggatggg gctcatggtg tttgccatgg gctggcgatc cttcaccaat gtcaactcca 2640
   ggatgctcta cttcgcccct gatctggttt tcaatgagta ccgcatgcac aagtcccgga 2700
   tgtacagcca gtgtgtccga atgaggcacc tctctcaaga gtttggatgg ctccaaatca 2760
   ccccccagga attcctgtgc atgaaagcac tgctactctt cagcattatt ccagtggatg 2820
   ggctgaaaaa tcaaaaattc tttgatgaac ttcgaatgaa ctacatcaag gaactcgatc 2880
   gtatcattgc atgcaaaaga aaaaatccca catcctgctc aagacgcttc taccagctca 2940
   ccaagctcct ggactccgtg cagcctattg cgagagagct gcatcagttc acttttgacc 3000
   tgctaatcaa gtcacacatg gtgagcgtgg actttccgga aatgatggca gagatcatct 3060
   ctgtgcaagt gcccaagatc ctttctggga aagtcaagcc catctatttc cacacccagt 3120
   gaagcattgg aaaccctatt tccccacccc agctcatgcc ccctttcaga tgtcttctgc 3180
   ctgttataac tctgcactac tcctctgcag tgccttgggg aatttcctct attgatgtac      3240
   agtctgtcat gaacatgttc ctgaattcta tttgctgggc tttttttttc tctttctctc      3300
   ctttcttttt cttcttccct ccctatctaa ccctcccatg gcaccttcag actttgcttc      3360
   ccattgtggc tcctatctgt gttttgaatg gtgttgtatg cctttaaatc tgtgatgatc      3420
   ctcatatggc ccagtgtcaa gttgtgcttg tttacagcac tactctgtgc cagccacaca      3480
   aacgtttact tatcttatgc cacgggaagt ttagagagct aagattatct ggggaaatca      3540
   aaacaaaaaa caagcaaaca aaaaaaaaa                                        3569
<210> 3
   <211> 648
   <212> PRT
   <213> Homo sapiens
<400> 3
   Met Glu Val Gln Leu Gly Leu Gly Arg Val Tyr Pro Arg Pro Pro Ser
   1               5                   10                  15
   Lys Thr Tyr Arg Gly Ala Phe Gln Asn Leu Phe Gln Ser Val Arg Glu
               20                  25                  30
   Val Ile Gln Asn Pro Gly Pro Arg His Pro Glu Ala Ala Ser Ala Ala
           35                  40                  45
   Pro Pro Gly Ala Ser Leu Leu Leu Gln Gln Gln Gln Gln Gln Gln Gln
       50                  55                  60
   Gln Gln Gln Gln Gln Gln Gln Gln Gln Gln Gln Gln Gln Gln Gln Gln
   65                  70                  75                  80
   Gln Gln Gln Gln Glu Thr Ser Pro Arg Gln Gln Gln Gln Gln Gln Gly
                   85                  90                  95
   Glu Asp Gly Ser Pro Gln Ala His Arg Arg Gly Pro Thr Gly Tyr Leu
               100                 105                 110
   Val Leu Asp Glu Glu Gln Gln Pro Ser Gln Pro Gln Ser Ala Leu Glu
           115                 120                 125
   Cys His Pro Glu Arg Gly Cys Val Pro Glu Pro Gly Ala Ala Val Ala
       130                 135                 140
   Ala Ser Lys Gly Leu Pro Gln Gln Leu Pro Ala Pro Pro Asp Glu Asp
   145                 150                 155                 160
   Asp Ser Ala Ala Pro Ser Thr Leu Ser Leu Leu Gly Pro Thr Phe Pro
                   165                 170                 175
   Gly Leu Ser Ser Cys Ser Ala Asp Leu Lys Asp Ile Leu Ser Glu Ala
               180                 185                 190
   Ser Thr Met Gln Leu Leu Gln Gln Gln Gln Gln Glu Ala Val Ser Glu
           195                 200                 205
   Gly Ser Ser Ser Gly Arg Ala Arg Glu Ala Ser Gly Ala Pro Thr Ser
       210                 215                 220
   Ser Lys Asp Asn Tyr Leu Gly Gly Thr Ser Thr Ile Ser Asp Asn Ala
   225                 230                 235                 240
   Lys Glu Leu Cys Lys Ala Val Ser Val Ser Met Gly Leu Gly Val Glu
                   245                 250                 255
   Ala Leu Glu His Leu Ser Pro Gly Glu Gln Leu Arg Gly Asp Cys Met
               260                 265                 270
   Tyr Ala Pro Leu Leu Gly Val Pro Pro Ala Val Arg Pro Thr Pro Cys
           275                 280                 285
   Ala Pro Leu Ala Glu Cys Lys Gly Ser Leu Leu Asp Asp Ser Ala Gly
       290                 295                 300
   Lys Ser Thr Glu Asp Thr Ala Glu Tyr Ser Pro Phe Lys Gly Gly Tyr
   305                 310                 315                 320
   Thr Lys Gly Leu Glu Gly Glu Ser Leu Gly Cys Ser Gly Ser Ala Ala
                   325                 330                 335
   Ala Gly Ser Ser Gly Thr Leu Glu Leu Pro Ser Thr Leu Ser Leu Tyr
               340                 345                 350
   Lys Ser Gly Ala Leu Asp Glu Ala Ala Ala Tyr Gln Ser Arg Asp Tyr
           355                 360                 365
   Tyr Asn Phe Pro Leu Ala Leu Ala Gly Pro Pro Pro Pro Pro Pro Pro
       370                 375                 380
   Pro His Pro His Ala Arg Ile Lys Leu Glu Asn Pro Leu Asp Tyr Gly
   385                 390                 395                 400
   Ser Ala Trp Ala Ala Ala Ala Ala Gln Cys Arg Tyr Gly Asp Leu Ala
                   405                 410                 415
   Ser Leu His Gly Ala Gly Ala Ala Gly Pro Gly Ser Gly Ser Pro Ser
               420                 425                 430
   Ala Ala Ala Ser Ser Ser Trp His Thr Leu Phe Thr Ala Glu Glu Gly
           435                 440                 445
   Gln Leu Tyr Gly Pro Cys Gly Gly Gly Gly Gly Gly Gly Gly Gly Gly
       450                 455                 460
   Gly Gly Gly Gly Gly Gly Gly Gly Gly Glu Ala Gly Ala Val Ala Pro
   465                 470                 475                 480
   Tyr Gly Tyr Thr Arg Pro Pro Gln Gly Leu Ala Gly Gln Glu Ser Asp
                   485                 490                 495
   Phe Thr Ala Pro Asp Val Trp Tyr Pro Gly Gly Met Val Ser Arg Val
               500                 505                 510
   Pro Tyr Pro Ser Pro Thr Cys Val Lys Ser Glu Met Gly Pro Trp Met
           515                 520                 525
   Asp Ser Tyr Ser Gly Pro Tyr Gly Asp Met Arg Leu Glu Thr Ala Arg
       530                 535                 540
   Asp His Val Leu Pro Ile Asp Tyr Tyr Phe Pro Pro Gln Lys Thr Cys
   545                 550                 555                 560
   Leu Ile Cys Gly Asp Glu Ala Ser Gly Cys His Tyr Gly Ala Leu Thr
                   565                 570                 575
   Cys Gly Ser Cys Lys Val Phe Phe Lys Arg Ala Ala Glu Gly Lys Gln
               580                 585                 590
   Lys Tyr Leu Cys Ala Ser Arg Asn Asp Cys Thr Ile Asp Lys Phe Arg
           595                 600                 605
   Arg Lys Asn Cys Pro Ser Cys Arg Leu Arg Lys Cys Tyr Glu Ala Gly
       610                 615                 620
   Met Thr Leu Gly Ala Ala Val Val Val Ser Glu Arg Ile Leu Arg Val
   625                 630                 635                 640
   Phe Gly Val Ser Glu Trp Leu Pro
                   645
<210> 4
   <211> 1968
   <212> DNA
   <213> Homo sapiens
<400> 4
   gatggaagtg cagttagggc tgggaagggt ctaccctcgg ccgccgtcca agacctaccg      60
   aggagctttc cagaatctgt tccagagcgt gcgcgaagtg atccagaacc cgggccccag     120
   gcacccagag gccgcgagcg cagcacctcc cggcgccagt ttgctgctgc agcagcagca     180
   gcagcagcag cagcagcagc agcagcagca gcagcagcag cagcagcagc agcagcagca     240
   gcagcagcag caagagacta gccccaggca gcagcagcag cagcagggtg aggatggttc     300
   tccccaagcc catcgtagag gccccacagg ctacctggtc ctggatgagg aacagcaacc     360
   ttcacagccg cagtcggccc tggagtgcca ccccgagaga ggttgcgtcc cagagcctgg     420
   agccgccgtg gccgccagca aggggctgcc gcagcagctg ccagcacctc cggacgagga 480
   tgactcagct gccccatcca cgttgtccct gctgggcccc actttccccg gcttaagcag 540
   ctgctccgct gaccttaaag acatcctgag cgaggccagc accatgcaac tccttcagca 600
   acagcagcag gaagcagtat ccgaaggcag cagcagcggg agagcgaggg aggcctcggg 660
   ggctcccact tcctccaagg acaattactt agggggcact tcgaccattt ctgacaacgc 720
   caaggagttg tgtaaggcag tgtcggtgtc catgggcctg ggtgtggagg cgttggagca 780
   tctgagtcca ggggaacagc ttcgggggga ttgcatgtac gccccacttt tgggagttcc 840
   acccgctgtg cgtcccactc cttgtgcccc attggccgaa tgcaaaggtt ctctgctaga 900
   cgacagcgca ggcaagagca ctgaagatac tgctgagtat tcccctttca agggaggtta 960
   caccaaaggg ctagaaggcg agagcctagg ctgctctggc agcgctgcag cagggagctc 1020
   cgggacactt gaactgccgt ctaccctgtc tctctacaag tccggagcac tggacgaggc 1080
   agctgcgtac cagagtcgcg actactacaa ctttccactg gctctggccg gaccgccgcc 1140
   ccctccgccg cctccccatc cccacgctcg catcaagctg gagaacccgc tggactacgg 1200
   cagcgcctgg gcggctgcgg cggcgcagtg ccgctatggg gacctggcga gcctgcatgg 1260
   cgcgggtgca gcgggacccg gttctgggtc accctcagcc gccgcttcct catcctggca 1320
   cactctcttc acagccgaag aaggccagtt gtatggaccg tgtggtggtg gtgggggtgg 1380
   tggcggcggc ggcggcggcg gcggcggcgg cggcggcggc gaggcgggag ctgtagcccc 1440
   ctacggctac actcggcccc ctcaggggct ggcgggccag gaaagcgact tcaccgcacc 1500
   tgatgtgtgg taccctggcg gcatggtgag cagagtgccc tatcccagtc ccacttgtgt 1560
   caaaagcgaa atgggcccct ggatggatag ctactccgga ccttacgggg acatgcgttt 1620
   ggagactgcc agggaccatg ttttgcccat tgactattac tttccacccc agaagacctg 1680
   cctgatctgt ggagatgaag cttctgggtg tcactatgga gctctcacat gtggaagctg 1740
   caaggtcttc ttcaaaagag ccgctgaagg gaaacagaag tacctgtgcg ccagcagaaa 1800
   tgattgcact attgataaat tccgaaggaa aaattgtcca tcttgtcgtc ttcggaaatg 1860
   ttatgaagca gggatgactc tgggagcagc tgttgttgtt tctgaaagaa tcttgagggt 1920
   gtttggagtc tcagaatggc ttccttaaag actaccttca gactctca 1968
<210> 5
   <211> 687
   <212> PRT
   <213> Homo sapiens
<400> 5
   Met Glu Val Gln Leu Gly Leu Gly Arg Val Tyr Pro Arg Pro Pro Ser
   1               5                   10                  15
   Lys Thr Tyr Arg Gly Ala Phe Gln Asn Leu Phe Gln Ser Val Arg Glu
               20                  25                  30
   Val Ile Gln Asn Pro Gly Pro Arg His Pro Glu Ala Ala Ser Ala Ala
           35                  40                  45
   Pro Pro Gly Ala Ser Leu Leu Leu Gln Gln Gln Gln Gln Gln Gln Gln
       50                  55                  60
   Gln Gln Gln Gln Gln Gln Gln Gln Gln Gln Gln Gln Gln Gln Gln Gln
   65                  70                  75                  80
   Gln Gln Gln Gln Glu Thr Ser Pro Arg Gln Gln Gln Gln Gln Gln Gly
                   85                  90                  95
   Glu Asp Gly Ser Pro Gln Ala His Arg Arg Gly Pro Thr Gly Tyr Leu
               100                 105                 110
   Val Leu Asp Glu Glu Gln Gln Pro Ser Gln Pro Gln Ser Ala Leu Glu
           115                 120                 125
   Cys His Pro Glu Arg Gly Cys Val Pro Glu Pro Gly Ala Ala Val Ala
       130                 135                 140
   Ala Ser Lys Gly Leu Pro Gln Gln Leu Pro Ala Pro Pro Asp Glu Asp
   145                 150                 155                 160
   Asp Ser Ala Ala Pro Ser Thr Leu Ser Leu Leu Gly Pro Thr Phe Pro
                   165                 170                 175
   Gly Leu Ser Ser Cys Ser Ala Asp Leu Lys Asp Ile Leu Ser Glu Ala
               180                 185                 190
   Ser Thr Met Gln Leu Leu Gln Gln Gln Gln Gln Glu Ala Val Ser Glu
           195                 200                 205
   Gly Ser Ser Ser Gly Arg Ala Arg Glu Ala Ser Gly Ala Pro Thr Ser
       210                 215                 220
   Ser Lys Asp Asn Tyr Leu Gly Gly Thr Ser Thr Ile Ser Asp Asn Ala
   225                 230                 235                 240
   Lys Glu Leu Cys Lys Ala Val Ser Val Ser Met Gly Leu Gly Val Glu
                   245                 250                 255
   Ala Leu Glu His Leu Ser Pro Gly Glu Gln Leu Arg Gly Asp Cys Met
               260                 265                 270
   Tyr Ala Pro Leu Leu Gly Val Pro Pro Ala Val Arg Pro Thr Pro Cys
           275                 280                 285
   Ala Pro Leu Ala Glu Cys Lys Gly Ser Leu Leu Asp Asp Ser Ala Gly
       290                 295                 300
   Lys Ser Thr Glu Asp Thr Ala Glu Tyr Ser Pro Phe Lys Gly Gly Tyr
   305                 310                 315                 320
   Thr Lys Gly Leu Glu Gly Glu Ser Leu Gly Cys Ser Gly Ser Ala Ala
                   325                 330                 335
   Ala Gly Ser Ser Gly Thr Leu Glu Leu Pro Ser Thr Leu Ser Leu Tyr
               340                 345                 350
   Lys Ser Gly Ala Leu Asp Glu Ala Ala Ala Tyr Gln Ser Arg Asp Tyr
           355                 360                 365
   Tyr Asn Phe Pro Leu Ala Leu Ala Gly Pro Pro Pro Pro Pro Pro Pro
       370                 375                 380
   Pro His Pro His Ala Arg Ile Lys Leu Glu Asn Pro Leu Asp Tyr Gly
   385                 390                 395                 400
   Ser Ala Trp Ala Ala Ala Ala Ala Gln Cys Arg Tyr Gly Asp Leu Ala
                   405                 410                 415
   Ser Leu His Gly Ala Gly Ala Ala Gly Pro Gly Ser Gly Ser Pro Ser
               420                 425                 430
   Ala Ala Ala Ser Ser Ser Trp His Thr Leu Phe Thr Ala Glu Glu Gly
           435                 440                 445
   Gln Leu Tyr Gly Pro Cys Gly Gly Gly Gly Gly Gly Gly Gly Gly Gly
       450                 455                 460
   Gly Gly Gly Gly Gly Gly Gly Gly Gly Glu Ala Gly Ala Val Ala Pro
   465                 470                 475                 480
   Tyr Gly Tyr Thr Arg Pro Pro Gln Gly Leu Ala Gly Gln Glu Ser Asp
                   485                 490                 495
   Phe Thr Ala Pro Asp Val Trp Tyr Pro Gly Gly Met Val Ser Arg Val
               500                 505                 510
   Pro Tyr Pro Ser Pro Thr Cys Val Lys Ser Glu Met Gly Pro Trp Met
               515                     520                     525
   Asp Ser Tyr Ser Gly Pro Tyr Gly Asp Met Arg Leu Glu Thr Ala Arg
       530                 535                 540
   Asp His Val Leu Pro Ile Asp Tyr Tyr Phe Pro Pro Gln Lys Thr Cys
   545                 550                 555                 560
   Leu Ile Cys Gly Asp Glu Ala Ser Gly Cys His Tyr Gly Ala Leu Thr
                   565                 570                 575
   Cys Gly Ser Cys Lys Val Phe Phe Lys Arg Ala Ala Glu Gly Lys Gln
               580                 585                 590
   Lys Tyr Leu Cys Ala Ser Arg Asn Asp Cys Thr Ile Asp Lys Phe Arg
           595                 600                 605
   Arg Lys Asn Cys Pro Ser Cys Arg Leu Arg Lys Cys Tyr Glu Ala Gly
       610                 615                 620
   Met Thr Leu Gly Gly Lys Gln Lys Tyr Leu Cys Ala Ser Arg Asn Asp
   625                 630                 635                 640
   Cys Thr Ile Asp Lys Phe Arg Arg Lys Asn Cys Pro Ser Cys Arg Leu
                   645                 650                 655
   Arg Lys Cys Tyr Glu Ala Gly Met Thr Leu Gly Ala Ala Val Val Val
               660                 665                 670
   Ser Glu Arg Ile Leu Arg Val Phe Gly Val Ser Glu Trp Leu Pro
           675                 680                 685
<210> 6
   <211> 2085
   <212> DNA
   <213> Homo sapiens
<400> 6
   gatggaagtg cagttagggc tgggaagggt ctaccctcgg ccgccgtcca agacctaccg 60
   aggagctttc cagaatctgt tccagagcgt gcgcgaagtg atccagaacc cgggccccag 120
   gcacccagag gccgcgagcg cagcacctcc cggcgccagt ttgctgctgc agcagcagca 180
   gcagcagcag cagcagcagc agcagcagca gcagcagcag cagcagcagc agcagcagca 240
   gcagcagcag caagagacta gccccaggca gcagcagcag cagcagggtg aggatggttc 300
   tccccaagcc catcgtagag gccccacagg ctacctggtc ctggatgagg aacagcaacc 360
   ttcacagccg cagtcggccc tggagtgcca ccccgagaga ggttgcgtcc cagagcctgg 420
   agccgccgtg gccgccagca aggggctgcc gcagcagctg ccagcacctc cggacgagga 480
   tgactcagct gccccatcca cgttgtccct gctgggcccc actttccccg gcttaagcag 540
   ctgctccgct gaccttaaag acatcctgag cgaggccagc accatgcaac tccttcagca 600
   acagcagcag gaagcagtat ccgaaggcag cagcagcggg agagcgaggg aggcctcggg 660
   ggctcccact tcctccaagg acaattactt agggggcact tcgaccattt ctgacaacgc 720
   caaggagttg tgtaaggcag tgtcggtgtc catgggcctg ggtgtggagg cgttggagca 780
   tctgagtcca ggggaacagc ttcgggggga ttgcatgtac gccccacttt tgggagttcc 840
   acccgctgtg cgtcccactc cttgtgcccc attggccgaa tgcaaaggtt ctctgctaga 900
   cgacagcgca ggcaagagca ctgaagatac tgctgagtat tcccctttca agggaggtta 960
   caccaaaggg ctagaaggcg agagcctagg ctgctctggc agcgctgcag cagggagctc 1020
   cgggacactt gaactgccgt ctaccctgtc tctctacaag tccggagcac tggacgaggc 1080
   agctgcgtac cagagtcgcg actactacaa ctttccactg gctctggccg gaccgccgcc 1140
   ccctccgccg cctccccatc cccacgctcg catcaagctg gagaacccgc tggactacgg 1200
   cagcgcctgg gcggctgcgg cggcgcagtg ccgctatggg gacctggcga gcctgcatgg 1260
   cgcgggtgca gcgggacccg gttctgggtc accctcagcc gccgcttcct catcctggca 1320
   cactctcttc acagccgaag aaggccagtt gtatggaccg tgtggtggtg gtgggggtgg 1380
   tggcggcggc ggcggcggcg gcggcggcgg cggcggcggc gaggcgggag ctgtagcccc 1440
   ctacggctac actcggcccc ctcaggggct ggcgggccag gaaagcgact tcaccgcacc 1500
   tgatgtgtgg taccctggcg gcatggtgag cagagtgccc tatcccagtc ccacttgtgt 1560
   caaaagcgaa atgggcccct ggatggatag ctactccgga ccttacgggg acatgcgttt 1620
   ggagactgcc agggaccatg ttttgcccat tgactattac tttccacccc agaagacctg 1680
   cctgatctgt ggagatgaag cttctgggtg tcactatgga gctctcacat gtggaagctg 1740
   caaggtcttc ttcaaaagag ccgctgaagg gaaacagaag tacctgtgcg ccagcagaaa 1800
   tgattgcact attgataaat tccgaaggaa aaattgtcca tcttgtcgtc ttcggaaatg 1860
   ttatgaagca gggatgactc tgggagggaa acagaagtac ctgtgcgcca gcagaaatga 1920
   ttgcactatt gataaattcc gaaggaaaaa ttgtccatct tgtcgtcttc ggaaatgtta 1980
   tgaagcaggg atgactctgg gagcagctgt tgttgtttct gaaagaatct tgagggtgtt 2040
   tggagtctca gaatggcttc cttaaagact accttcagac tctca 2085
<210> 7
   <211> 643
   <212> PRT
   <213> Homo sapiens
<400> 7
   Met Glu Val Gln Leu Gly Leu Gly Arg Val Tyr Pro Arg Pro Pro Ser
   1               5                   10                  15
   Lys Thr Tyr Arg Gly Ala Phe Gln Asn Leu Phe Gln Ser Val Arg Glu
               20                  25                  30
   Val Ile Gln Asn Pro Gly Pro Arg His Pro Glu Ala Ala Ser Ala Ala
           35                  40                  45
   Pro Pro Gly Ala Ser Leu Leu Leu Gln Gln Gln Gln Gln Gln Gln Gln
       50                  55                  60
   Gln Gln Gln Gln Gln Gln Gln Gln Gln Gln Gln Gln Gln Gln Gln Gln
   65                  70                  75                  80
   Gln Gln Gln Gln Gln Glu Thr Ser Pro Arg Gln Gln Gln Gln Gln Gln
                   85                  90                  95
   Gly Glu Asp Gly Ser Pro Gln Ala His Arg Arg Gly Pro Thr Gly Tyr
               100                 105                 110
   Leu Val Leu Asp Glu Glu Gln Gln Pro Ser Gln Pro Gln Ser Ala Leu
           115                 120                 125
   Glu Cys His Pro Glu Arg Gly Cys Val Pro Glu Pro Gly Ala Ala Val
       130                 135                 140
   Ala Ala Ser Lys Gly Leu Pro Gln Gln Leu Pro Ala Pro Pro Asp Glu
   145                 150                 155                 160
   Asp Asp Ser Ala Ala Pro Ser Thr Leu Ser Leu Leu Gly Pro Thr Phe
                   165                 170                 175
   Pro Gly Leu Ser Ser Cys Ser Ala Asp Leu Lys Asp Ile Leu Ser Glu
               180                 185                 190
   Ala Ser Thr Met Gln Leu Leu Gln Gln Gln Gln Gln Glu Ala Val Ser
           195                 200                 205
   Glu Gly Ser Ser Ser Gly Arg Ala Arg Glu Ala Ser Gly Ala Pro Thr
       210                 215                 220
   Ser Ser Lys Asp Asn Tyr Leu Gly Gly Thr Ser Thr Ile Ser Asp Asn
   225                 230                 235                 240
   Ala Lys Glu Leu Cys Lys Ala Val Ser Val Ser Met Gly Leu Gly Val
                   245                 250                 255
   Glu Ala Leu Glu His Leu Ser Pro Gly Glu Gln Leu Arg Gly Asp Cys
               260                 265                 270
   Met Tyr Ala Pro Leu Leu Gly Val Pro Pro Ala Val Arg Pro Thr Pro
           275                 280                 285
   Cys Ala Pro Leu Ala Glu Cys Lys Gly Ser Leu Leu Asp Asp Ser Ala
       290                 295                 300
   Gly Lys Ser Thr Glu Asp Thr Ala Glu Tyr Ser Pro Phe Lys Gly Gly
   305                 310                 315                 320
   Tyr Thr Lys Gly Leu Glu Gly Glu Ser Leu Gly Cys Ser Gly Ser Ala
                   325                 330                 335
   Ala Ala Gly Ser Ser Gly Thr Leu Glu Leu Pro Ser Thr Leu Ser Leu
               340                 345                 350
   Tyr Lys Ser Gly Ala Leu Asp Glu Ala Ala Ala Tyr Gln Ser Arg Asp
           355                 360                 365
   Tyr Tyr Asn Phe Pro Leu Ala Leu Ala Gly Pro Pro Pro Pro Pro Pro
       370                 375                 380
   Pro Pro His Pro His Ala Arg Ile Lys Leu Glu Asn Pro Leu Asp Tyr
   385                 390                 395                 400
   Gly Ser Ala Trp Ala Ala Ala Ala Ala Gln Cys Arg Tyr Gly Asp Leu
                   405                 410                 415
   Ala Ser Leu His Gly Ala Gly Ala Ala Gly Pro Gly Ser Gly Ser Pro
               420                 425                 430
   Ser Ala Ala Ala Ser Ser Ser Trp His Thr Leu Phe Thr Ala Glu Glu
           435                 440                 445
   Gly Gln Leu Tyr Gly Pro Cys Gly Gly Gly Gly Gly Gly Gly Gly Gly
       450                 455                 460
   Gly Gly Gly Gly Gly Gly Gly Gly Gly Gly Glu Ala Gly Ala Val Ala
   465                 470                 475                 480
   Pro Tyr Gly Tyr Thr Arg Pro Pro Gln Gly Leu Ala Gly Gln Glu Ser
                   485                 490                 495
   Asp Phe Thr Ala Pro Asp Val Trp Tyr Pro Gly Gly Met Val Ser Arg
               500                 505                 510
   Val Pro Tyr Pro Ser Pro Thr Cys Val Lys Ser Glu Met Gly Pro Trp
           515                 520                 525
   Met Asp Ser Tyr Ser Gly Pro Tyr Gly Asp Met Arg Leu Glu Thr Ala
       530                 535                 540
   Arg Asp His Val Leu Pro Ile Asp Tyr Tyr Phe Pro Pro Gln Lys Thr
   545                 550                 555                 560
   Cys Leu Ile Cys Gly Asp Glu Ala Ser Gly Cys His Tyr Gly Ala Leu
                   565                 570                 575
   Thr Cys Gly Ser Cys Lys Val Phe Phe Lys Arg Ala Ala Glu Gly Phe
               580                 585                 590
   Phe Arg Met Asn Lys Leu Lys Glu Ser Ser Asp Thr Asn Pro Lys Pro
           595                 600                 605
   Tyr Cys Met Ala Ala Pro Met Gly Leu Thr Glu Asn Asn Arg Asn Arg
       610                 615                 620
   Lys Lys Ser Tyr Arg Glu Thr Asn Leu Lys Ala Val Ser Trp Pro Leu
   625                 630                 635                 640
   Asn His Thr
<210> 8
   <211> 1934
   <212> DNA
   <213> Homo sapiens
<400> 8
   gatggaagtg cagttagggc tgggaagggt ctaccctcgg ccgccgtcca agacctaccg 60
   aggagctttc cagaatctgt tccagagcgt gcgcgaagtg atccagaacc cgggccccag 120
   gcacccagag gccgcgagcg cagcacctcc cggcgccagt ttgctgctgc agcagcagca 180
   gcagcagcag cagcagcagc agcagcagca gcagcagcag cagcagcagc agcagcagca 240
   gcagcagcag cagcaagaga ctagccccag gcagcagcag cagcagcagg gtgaggatgg 300
   ttctccccaa gcccatcgta gaggccccac aggctacctg gtcctggatg aggaacagca 360
   accttcacag ccgcagtcgg ccctggagtg ccaccccgag agaggttgcg tcccagagcc 420
   tggagccgcc gtggccgcca gcaaggggct gccgcagcag ctgccagcac ctccggacga 480
   ggatgactca gctgccccat ccacgttgtc cctgctgggc cccactttcc ccggcttaag 540
   cagctgctcc gctgacctta aagacatcct gagcgaggcc agcaccatgc aactccttca 600
   gcaacagcag caggaagcag tatccgaagg cagcagcagc gggagagcga gggaggcctc 660
   gggggctccc acttcctcca aggacaatta cttagggggc acttcgacca tttctgacaa 720
   cgccaaggag ttgtgtaagg cagtgtcggt gtccatgggc ctgggtgtgg aggcgttgga 780
   gcatctgagt ccaggggaac agcttcgggg ggattgcatg tacgccccac ttttgggagt 840
   tccacccgct gtgcgtccca ctccttgtgc cccattggcc gaatgcaaag gttctctgct 900
   agacgacagc gcaggcaaga gcactgaaga tactgctgag tattcccctt tcaagggagg 960
   ttacaccaaa gggctagaag gcgagagcct aggctgctct ggcagcgctg cagcagggag 1020
   ctccgggaca cttgaactgc cgtctaccct gtctctctac aagtccggag cactggacga 1080
   ggcagctgcg taccagagtc gcgactacta caactttcca ctggctctgg ccggaccgcc 1140
   gccccctccg ccgcctcccc atccccacgc tcgcatcaag ctggagaacc cgctggacta 1200
   cggcagcgcc tgggcggctg cggcggcgca gtgccgctat ggggacctgg cgagcctgca 1260
   tggcgcgggt gcagcgggac ccggttctgg gtcaccctca gccgccgctt cctcatcctg 1320
   gcacactctc ttcacagccg aagaaggcca gttgtatgga ccgtgtggtg gtggtggggg 1380
   tggtggcggc ggcggcggcg gcggcggcgg cggcggcggc ggcgaggcgg gagctgtagc 1440
   cccctacggc tacactcggc cccctcaggg gctggcgggc caggaaagcg acttcaccgc 1500
   acctgatgtg tggtaccctg gcggcatggt gagcagagtg ccctatccca gtcccacttg 1560
   tgtcaaaagc gaaatgggcc cctggatgga tagctactcc ggaccttacg gggacatgcg 1620
   tttggagact gccagggacc atgttttgcc cattgactat tactttccac cccagaagac    1680
   ctgcctgatc tgtggagacg aagcttctgg gtgtcactat ggagctctca catgtggaag 1740
   ctgcaaggtc ttcttcaaaa gagccgctga aggatttttc agaatgaaca aattaaaaga 1800
   atcatcagac actaacccca agccatactg catggcagca ccaatgggac tgacagaaaa 1860
   caacagaaat aggaagaaat cctacagaga aacaaacttg aaagctgtct catggccttt 1920
   gaatcatact taag 1934
<210> 9
   <211> 629
   <212> PRT
   <213> Homo sapiens
<400> 9
   Met Glu Val Gln Leu Gly Leu Gly Arg Val Tyr Pro Arg Pro Pro Ser
   1               5                   10                  15
   Lys Thr Tyr Arg Gly Ala Phe Gln Asn Leu Phe Gln Ser Val Arg Glu
               20                  25                  30
   Val Ile Gln Asn Pro Gly Pro Arg His Pro Glu Ala Ala Ser Ala Ala
           35                  40                  45
   Pro Pro Gly Ala Ser Leu Leu Leu Leu Gln Gln Gln Gln Gln Gln Gln
       50                  55                  60
   Gln Gln Gln Gln Gln Gln Gln Gln Gln Gln Gln Gln Gln Gln Gln Gln
   65                  70                  75                  80
   Glu Thr Ser Pro Arg Gln Gln Gln Gln Gln Gln Gly Glu Asp Gly Ser
                   85                  90                  95
   Pro Gln Ala His Arg Arg Gly Pro Thr Gly Tyr Leu Val Leu Asp Glu
               100                 105                 110
   Glu Gln Gln Pro Ser Gln Pro Gln Ser Ala Leu Glu Cys His Pro Glu
           115                 120                 125
   Arg Gly Cys Val Pro Glu Pro Gly Ala Ala Val Ala Ala Ser Lys Gly
       130                 135                 140
   Leu Pro Gln Gln Leu Pro Ala Pro Pro Asp Glu Asp Asp Ser Ala Ala
   145                 150                 155                 160
   Pro Ser Thr Leu Ser Leu Leu Gly Pro Thr Phe Pro Gly Leu Ser Ser
                   165                 170                 175
   Cys Ser Ala Asp Leu Lys Asp Ile Leu Ser Glu Ala Ser Thr Met Gln
               180                 185                 190
   Leu Leu Gln Gln Gln Gln Gln Glu Ala Val Ser Glu Gly Ser Ser Ser
           195                 200                 205
   Gly Arg Ala Arg Glu Ala Ser Gly Ala Pro Thr Ser Ser Lys Asp Asn
       210                 215                 220
   Tyr Leu Gly Gly Thr Ser Thr Ile Ser Asp Asn Ala Lys Glu Leu Cys
   225                 230                 235                 240
   Lys Ala Val Ser Val Ser Met Gly Leu Gly Val Glu Ala Leu Glu His
                   245                 250                 255
   Leu Ser Pro Gly Glu Gln Leu Arg Gly Asp Cys Met Tyr Ala Pro Leu
               260                 265                 270
   Leu Gly Val Pro Pro Ala Val Arg Pro Thr Pro Cys Ala Pro Leu Ala
           275                 280                 285
   Glu Cys Lys Gly Ser Leu Leu Asp Asp Ser Ala Gly Lys Ser Thr Glu
       290                 295                 300
   Asp Thr Ala Glu Tyr Ser Pro Phe Lys Gly Gly Tyr Thr Lys Gly Leu
   305                 310                 315                 320
   Glu Gly Glu Ser Leu Gly Cys Ser Gly Ser Ala Ala Ala Gly Ser Ser
                   325                 330                 335
   Gly Thr Leu Glu Leu Pro Ser Thr Leu Ser Leu Tyr Lys Ser Gly Ala
               340                 345                 350
   Leu Asp Glu Ala Ala Ala Tyr Gln Ser Arg Asp Tyr Tyr Asn Phe Pro
           355                 360                 365
   Leu Ala Leu Ala Gly Pro Pro Pro Pro Pro Pro Pro Pro His Pro His
       370                 375                 380
   Ala Arg Ile Lys Leu Glu Asn Pro Leu Asp Tyr Gly Ser Ala Trp Ala
   385                 390                 395                 400
   Ala Ala Ala Ala Gln Cys Arg Tyr Gly Asp Leu Ala Ser Leu His Gly
                   405                 410                 415
   Ala Gly Ala Ala Gly Pro Gly Ser Gly Ser Pro Ser Ala Ala Ala Ser
               420                 425                 430
   Ser Ser Trp His Thr Leu Phe Thr Ala Glu Glu Gly Gln Leu Tyr Gly
           435                 440                 445
   Pro Cys Gly Gly Gly Gly Gly Gly Gly Gly Gly Gly Gly Gly Gly Gly
       450                 455                 460
   Gly Gly Gly Gly Gly Gly Gly Gly Gly Glu Ala Gly Ala Val Ala Pro
   465                 470                 475                 480
   Tyr Gly Tyr Thr Arg Pro Pro Gln Gly Leu Ala Gly Gln Glu Ser Asp
                   485                 490                 495
   Phe Thr Ala Pro Asp Val Trp Tyr Pro Gly Gly Met Val Ser Arg Val
               500                 505                 510
   Pro Tyr Pro Ser Pro Thr Cys Val Lys Ser Glu Met Gly Pro Trp Met
           515                 520                 525
   Asp Ser Tyr Ser Gly Pro Tyr Gly Asp Met Arg Leu Glu Thr Ala Arg
       530                 535                 540
   Asp His Val Leu Pro Ile Asp Tyr Tyr Phe Pro Pro Gln Lys Thr Cys
   545                 550                 555                 560
   Leu Ile Cys Gly Asp Glu Ala Ser Gly Cys His Tyr Gly Ala Leu Thr
                   565                 570                 575
   Cys Gly Ser Cys Lys Val Phe Phe Lys Arg Ala Ala Glu Gly Lys Gln
               580                 585                 590
   Lys Tyr Leu Cys Ala Ser Arg Asn Asp Cys Thr Ile Asp Lys Phe Arg
           595                 600                 605
   Arg Lys Asn Cys Pro Ser Cys Arg Leu Arg Lys Cys Tyr Glu Ala Gly
       610                 615                 620
   Met Thr Leu Gly Asp
   625
<210> 10
   <211> 1892
   <212> DNA
   <213> Homo sapiens
<400> 10
   gatggaagtg cagttagggc tgggaagggt ctaccctcgg ccgccgtcca agacctaccg 60
   aggagctttc cagaatctgt tccagagcgt gcgcgaagtg atccagaacc cgggccccag 120
   gcacccagag gccgcgagcg cagcacctcc cggcgccagt ttgctgctgc tgcagcagca 180
   gcagcagcag cagcagcagc agcagcagca gcagcagcag cagcagcagc agcagcagca 240
   agagactagc cccaggcagc agcagcagca gcagggtgag gatggttctc cccaagccca 300
   tcgtagaggc cccacaggct acctggtcct ggatgaggaa cagcaacctt cacagccgca 360
   gtcggccctg gagtgccacc ccgagagagg ttgcgtccca gagcctggag ccgccgtggc 420
   cgccagcaag gggctgccgc agcagctgcc agcacctccg gacgaggatg actcagctgc 480
   cccatccacg ttgtccctgc tgggccccac tttccccggc ttaagcagct gctccgctga 540
   ccttaaagac atcctgagcg aggccagcac catgcaactc cttcagcaac agcagcagga 600
   agcagtatcc gaaggcagca gcagcgggag agcgagggag gcctcggggg ctcccacttc 660
   ctccaaggac aattacttag ggggcacttc gaccatttct gacaacgcca aggagttgtg 720
   taaggcagtg tcggtgtcca tgggcctggg tgtggaggcg ttggagcatc tgagtccagg 780
   ggaacagctt cggggggatt gcatgtacgc cccacttttg ggagttccac ccgctgtgcg 840
   tcccactcct tgtgccccat tggccgaatg caaaggttct ctgctagacg acagcgcagg 900
   caagagcact gaagatactg ctgagtattc ccctttcaag ggaggttaca ccaaagggct 960
   agaaggcgag agcctaggct gctctggcag cgctgcagca gggagctccg ggacacttga 1020
   actgccgtct accctgtctc tctacaagtc cggagcactg gacgaggcag ctgcgtacca 1080
   gagtcgcgac tactacaact ttccactggc tctggccgga ccgccgcccc ctccgccgcc 1140
   tccccatccc cacgctcgca tcaagctgga gaacccgctg gactacggca gcgcctgggc 1200
   ggctgcggcg gcgcagtgcc gctatgggga cctggcgagc ctgcatggcg cgggtgcagc 1260
   gggacccggt tctgggtcac cctcagccgc cgcttcctca tcctggcaca ctctcttcac 1320
   agccgaagaa ggccagttgt atggaccgtg tggtggtggt gggggtggtg gcggcggcgg 1380
   cggcggcggc ggcggcggcg gcggcggcgg cggcggcggc gaggcgggag ctgtagcccc 1440
   ctacggctac actcggcccc ctcaggggct ggcgggccag gaaagcgact tcaccgcacc 1500
   tgatgtgtgg taccctggcg gcatggtgag cagagtgccc tatcccagtc ccacttgtgt 1560
   caaaagcgaa atgggcccct ggatggatag ctactccgga ccttacgggg acatgcgttt 1620
   ggagactgcc agggaccatg ttttgcccat tgactattac tttccacccc agaagacctg 1680
   cctgatctgt ggagatgaag cttctgggtg tcactatgga gctctcacat gtggaagctg 1740
   caaggtcttc ttcaaaagag ccgctgaagg gaaacagaag tacctgtgcg ccagcagaaa 1800
   tgattgcact attgataaat tccgaaggaa aaattgtcca tcttgtcgtc ttcggaaatg 1860
   ttatgaagca gggatgactc tgggagacta ga 1892
<210> 11
   <211> 645
   <212> PRT
   <213> Homo sapiens
<400> 11
   Met Glu Val Gln Leu Gly Leu Gly Arg Val Tyr Pro Arg Pro Pro Ser
   1               5                   10                  15
   Lys Thr Tyr Arg Gly Ala Phe Gln Asn Leu Phe Gln Ser Val Arg Glu
               20                  25                  30
   Val Ile Gln Asn Pro Gly Pro Arg His Pro Glu Ala Ala Ser Ala Ala
           35                  40                  45
   Pro Pro Gly Ala Ser Leu Leu Leu Gln Gln Gln Gln Gln Gln Gln Gln
       50                  55                  60
   Gln Gln Gln Gln Gln Gln Gln Gln Gln Gln Gln Gln Gln Gln Gln Gln
   65                  70                  75                  80
   Gln Gln Gln Gln Gln Glu Thr Ser Pro Arg Gln Gln Gln Gln Gln Gln
                   85                  90                  95
   Gly Glu Asp Gly Ser Pro Gln Ala His Arg Arg Gly Pro Thr Gly Tyr
               100                 105                 110
   Leu Val Leu Asp Glu Glu Gln Gln Pro Ser Gln Pro Gln Ser Ala Leu
           115                 120                 125
   Glu Cys His Pro Glu Arg Gly Cys Val Pro Glu Pro Gly Ala Ala Val
       130                 135                 140
   Ala Ala Ser Lys Gly Leu Pro Gln Gln Leu Pro Ala Pro Pro Asp Glu
   145                 150                 155                 160
   Asp Asp Ser Ala Ala Pro Ser Thr Leu Ser Leu Leu Gly Pro Thr Phe
                   165                 170                 175
   Pro Gly Leu Ser Ser Cys Ser Ala Asp Leu Lys Asp Ile Leu Ser Glu
               180                 185                 190
   Ala Ser Thr Met Gln Leu Leu Gln Gln Gln Gln Gln Glu Ala Val Ser
           195                 200                 205
   Glu Gly Ser Ser Ser Gly Arg Ala Arg Glu Ala Ser Gly Ala Pro Thr
       210                 215                 220
   Ser Ser Lys Asp Asn Tyr Leu Gly Gly Thr Ser Thr Ile Ser Asp Asn
   225                 230                 235                 240
   Ala Lys Glu Leu Cys Lys Ala Val Ser Val Ser Met Gly Leu Gly Val
                   245                 250                 255
   Glu Ala Leu Glu His Leu Ser Pro Gly Glu Gln Leu Arg Gly Asp Cys
               260                 265                 270
   Met Tyr Ala Pro Leu Leu Gly Val Pro Pro Ala Val Arg Pro Thr Pro
           275                 280                 285
   Cys Ala Pro Leu Ala Glu Cys Lys Gly Ser Leu Leu Asp Asp Ser Ala
       290                 295                 300
   Gly Lys Ser Thr Glu Asp Thr Ala Glu Tyr Ser Pro Phe Lys Gly Gly
   305                 310                 315                 320
   Tyr Thr Lys Gly Leu Glu Gly Glu Ser Leu Gly Cys Ser Gly Ser Ala
                   325                 330                 335
   Ala Ala Gly Ser Ser Gly Thr Leu Glu Leu Pro Ser Thr Leu Ser Leu
               340                 345                 350
   Tyr Lys Ser Gly Ala Leu Asp Glu Ala Ala Ala Tyr Gln Ser Arg Asp
           355                 360                 365
   Tyr Tyr Asn Phe Pro Leu Ala Leu Ala Gly Pro Pro Pro Pro Pro Pro
       370                 375                 380
   Pro Pro His Pro His Ala Arg Ile Lys Leu Glu Asn Pro Leu Asp Tyr
   385                 390                 395                 400
   Gly Ser Ala Trp Ala Ala Ala Ala Ala Gln Cys Arg Tyr Gly Asp Leu
                   405                 410                 415
   Ala Ser Leu His Gly Ala Gly Ala Ala Gly Pro Gly Ser Gly Ser Pro
               420                 425                 430
   Ser Ala Ala Ala Ser Ser Ser Trp His Thr Leu Phe Thr Ala Glu Glu
           435                 440                 445
   Gly Gln Leu Tyr Gly Pro Cys Gly Gly Gly Gly Gly Gly Gly Gly Gly
       450                 455                 460
   Gly Gly Gly Gly Gly Gly Gly Gly Gly Gly Glu Ala Gly Ala Val Ala
   465                 470                 475                 480
   Pro Tyr Gly Tyr Thr Arg Pro Pro Gln Gly Leu Ala Gly Gln Glu Ser
                   485                 490                 495
   Asp Phe Thr Ala Pro Asp Val Trp Tyr Pro Gly Gly Met Val Ser Arg
               500                 505                 510
   Val Pro Tyr Pro Ser Pro Thr Cys Val Lys Ser Glu Met Gly Pro Trp
           515                 520                 525
   Met Asp Ser Tyr Ser Gly Pro Tyr Gly Asp Met Arg Leu Glu Thr Ala
       530                 535                 540
   Arg Asp His Val Leu Pro Ile Asp Tyr Tyr Phe Pro Pro Gln Lys Thr
   545                 550                 555                 560
   Cys Leu Ile Cys Gly Asp Glu Ala Ser Gly Cys His Tyr Gly Ala Leu
                   565                 570                 575
   Thr Cys Gly Ser Cys Lys Val Phe Phe Lys Arg Ala Ala Glu Gly Lys
                           580                     585                     590
   Gln Lys Tyr Leu Cys Ala Ser Arg Asn Asp Cys Thr Ile Asp Lys Phe
                   595                     600                     605
   Arg Arg Lys Asn Cys Pro Ser Cys Arg Leu Arg Lys Cys Tyr Glu Ala
       610                     615                     620
   Gly Met Thr Leu Gly Glu Lys Phe Arg Val Gly Asn Cys Lys His Leu
   625                     630                     635                     640
   Lys Met Thr Arg Pro
                   645
<210> 12
   <211> 1939
   <212> DNA
   <213> Homo sapiens
<400> 12
   gatggaagtg cagttagggc tgggaagggt ctaccctcgg ccgccgtcca agacctaccg 60
   aggagctttc cagaatctgt tccagagcgt gcgcgaagtg atccagaacc cgggccccag 120
   gcacccagag gccgcgagcg cagcacctcc cggcgccagt ttgctgctgc agcagcagca 180
   gcagcagcag cagcagcagc agcagcagca gcagcagcag cagcagcagc agcagcagca 240
   gcagcagcag cagcaagaga ctagccccag gcagcagcag cagcagcagg gtgaggatgg 300
   ttctccccaa gcccatcgta gaggccccac aggctacctg gtcctggatg aggaacagca 360
   accttcacag ccgcagtcgg ccctggagtg ccaccccgag agaggttgcg tcccagagcc 420
   tggagccgcc gtggccgcca gcaaggggct gccgcagcag ctgccagcac ctccggacga 480
   ggatgactca gctgccccat ccacgttgtc cctgctgggc cccactttcc ccggcttaag 540
   cagctgctcc gctgacctta aagacatcct gagcgaggcc agcaccatgc aactccttca 600
   gcaacagcag caggaagcag tatccgaagg cagcagcagc gggagagcga gggaggcctc 660
   gggggctccc acttcctcca aggacaatta cttagggggc acttcgacca tttctgacaa 720
   cgccaaggag ttgtgtaagg cagtgtcggt gtccatgggc ctgggtgtgg aggcgttgga 780
   gcatctgagt ccaggggaac agcttcgggg ggattgcatg tacgccccac ttttgggagt 840
   tccacccgct gtgcgtccca ctccttgtgc cccattggcc gaatgcaaag gttctctgct 900
   agacgacagc gcaggcaaga gcactgaaga tactgctgag tattcccctt tcaagggagg 960
   ttacaccaaa gggctagaag gcgagagcct aggctgctct ggcagcgctg cagcagggag 1020
   ctccgggaca cttgaactgc cgtctaccct gtctctctac aagtccggag cactggacga 1080
   ggcagctgcg taccagagtc gcgactacta caactttcca ctggctctgg ccggaccgcc 1140
   gccccctccg ccgcctcccc atccccacgc tcgcatcaag ctggagaacc cgctggacta 1200
   cggcagcgcc tgggcggctg cggcggcgca gtgccgctat ggggacctgg cgagcctgca 1260
   tggcgcgggt gcagcgggac ccggttctgg gtcaccctca gccgccgctt cctcatcctg 1320
   gcacactctc ttcacagccg aagaaggcca gttgtatgga ccgtgtggtg gtggtggggg 1380
   tggtggcggc ggcggcggcg gcggcggcgg cggcggcggc ggcgaggcgg gagctgtagc    1440
   cccctacggc tacactcggc cccctcaggg gctggcgggc caggaaagcg acttcaccgc    1500
   acctgatgtg tggtaccctg gcggcatggt gagcagagtg ccctatccca gtcccacttg    1560
   tgtcaaaagc gaaatgggcc cctggatgga tagctactcc ggaccttacg gggacatgcg    1620
   tttggagact gccagggacc atgttttgcc cattgactat tactttccac cccagaagac    1680
   ctgcctgatc tgtggagatg aagcttctgg gtgtcactat ggagctctca catgtggaag    1740
   ctgcaaggtc ttcttcaaaa gagccgctga agggaaacag aagtacctgt gcgccagcag    1800
   aaatgattgc actattgata aattccgaag gaaaaattgt ccatcttgtc gtcttcggaa    1860
   atgttatgaa gcagggatga ctctgggaga aaaattccgg gttggcaatt gcaagcatct    1920
   caaaatgacc agaccctga                                                 1939
<210> 13
   <211> 651
   <212> PRT
   <213> Homo sapiens
<400> 13
   Met Glu Val Gln Leu Gly Leu Gly Arg Val Tyr Pro Arg Pro Pro Ser
   1               5                   10                  15
   Lys Thr Tyr Arg Gly Ala Phe Gln Asn Leu Phe Gln Ser Val Arg Glu
               20                  25                  30
   Val Ile Gln Asn Pro Gly Pro Arg His Pro Glu Ala Ala Ser Ala Ala
           35                  40                  45
   Pro Pro Gly Ala Ser Leu Leu Leu Leu Gln Gln Gln Gln Gln Gln Gln
       50                  55                  60
   Gln Gln Gln Gln Gln Gln Gln Gln Gln Gln Gln Gln Gln Gln Gln Gln
   65                  70                  75                  80
   Glu Thr Ser Pro Arg Gln Gln Gln Gln Gln Gln Gly Glu Asp Gly Ser
                   85                  90                  95
   Pro Gln Ala His Arg Arg Gly Pro Thr Gly Tyr Leu Val Leu Asp Glu
               100                 105                 110
   Glu Gln Gln Pro Ser Gln Pro Gln Ser Ala Leu Glu Cys His Pro Glu
           115                 120                 125
   Arg Gly Cys Val Pro Glu Pro Gly Ala Ala Val Ala Ala Ser Lys Gly
       130                 135                 140
   Leu Pro Gln Gln Leu Pro Ala Pro Pro Asp Glu Asp Asp Ser Ala Ala
   145                 150                 155                 160
   Pro Ser Thr Leu Ser Leu Leu Gly Pro Thr Phe Pro Gly Leu Ser Ser
                   165                 170                 175
   Cys Ser Ala Asp Leu Lys Asp Ile Leu Ser Glu Ala Ser Thr Met Gln
               180                 185                 190
   Leu Leu Gln Gln Gln Gln Gln Glu Ala Val Ser Glu Gly Ser Ser Ser
           195                 200                 205
   Gly Arg Ala Arg Glu Ala Ser Gly Ala Pro Thr Ser Ser Lys Asp Asn
       210                 215                 220
   Tyr Leu Gly Gly Thr Ser Thr Ile Ser Asp Asn Ala Lys Glu Leu Cys
   225                 230                 235                 240
   Lys Ala Val Ser Val Ser Met Gly Leu Gly Val Glu Ala Leu Glu His
                   245                 250                 255
   Leu Ser Pro Gly Glu Gln Leu Arg Gly Asp Cys Met Tyr Ala Pro Leu
               260                 265                 270
   Leu Gly Val Pro Pro Ala Val Arg Pro Thr Pro Cys Ala Pro Leu Ala
   275 280 285
   Glu Cys Lys Gly Ser Leu Leu Asp Asp Ser Ala Gly Lys Ser Thr Glu
   290 295 300
   Asp Thr Ala Glu Tyr Ser Pro Phe Lys Gly Gly Tyr Thr Lys Gly Leu
   305 310 315 320
   Glu Gly Glu Ser Leu Gly Cys Ser Gly Ser Ala Ala Ala Gly Ser Ser
   325 330 335
   Gly Thr Leu Glu Leu Pro Ser Thr Leu Ser Leu Tyr Lys Ser Gly Ala
   340 345 350
   Leu Asp Glu Ala Ala Ala Tyr Gln Ser Arg Asp Tyr Tyr Asn Phe Pro
   355 360 365
   Leu Ala Leu Ala Gly Pro Pro Pro Pro Pro Pro Pro Pro His Pro His
   370 375 380
   Ala Arg Ile Lys Leu Glu Asn Pro Leu Asp Tyr Gly Ser Ala Trp Ala
   385 390 395 400
   Ala Ala Ala Ala Gln Cys Arg Tyr Gly Asp Leu Ala Ser Leu His Gly
   405 410 415
   Ala Gly Ala Ala Gly Pro Gly Ser Gly Ser Pro Ser Ala Ala Ala Ser
   420 425 430
   Ser Ser Trp His Thr Leu Phe Thr Ala Glu Glu Gly Gln Leu Tyr Gly
   435 440 445
   Pro Cys Gly Gly Gly Gly Gly Gly Gly Gly Gly Gly Gly Gly Gly Gly
   450 455 460
   Gly Gly Gly Gly Gly Gly Gly Gly Gly Glu Ala Gly Ala Val Ala Pro
   465 470 475 480
   Tyr Gly Tyr Thr Arg Pro Pro Gln Gly Leu Ala Gly Gln Glu Ser Asp
   485 490 495
   Phe Thr Ala Pro Asp Val Trp Tyr Pro Gly Gly Met Val Ser Arg Val
   500 505 510
   Pro Tyr Pro Ser Pro Thr Cys Val Lys Ser Glu Met Gly Pro Trp Met
   515 520 525
   Asp Ser Tyr Ser Gly Pro Tyr Gly Asp Met Arg Leu Glu Thr Ala Arg
   530 535 540
   Asp His Val Leu Pro Ile Asp Tyr Tyr Phe Pro Pro Gln Lys Thr Cys
   545 550 555 560
   Leu Ile Cys Gly Asp Glu Ala Ser Gly Cys His Tyr Gly Ala Leu Thr
   565 570 575
   Cys Gly Ser Cys Lys Val Phe Phe Lys Arg Ala Ala Glu Gly Lys Gln
   580 585 590
   Lys Tyr Leu Cys Ala Ser Arg Asn Asp Cys Thr Ile Asp Lys Phe Arg
   595 600 605
   Arg Lys Asn Cys Pro Ser Cys Arg Leu Arg Lys Cys Tyr Glu Ala Gly
   610 615 620
   Met Thr Leu Gly Asp Asn Leu Pro Glu Gln Ala Ala Phe Trp Arg His
   625 630 635 640
   Leu His Ile Phe Trp Asp His Val Val Lys Lys
   645 650
<210> 14
   <211> 1957
   <212> DNA
   <213> Homo sapiens
<400> 14
   gatggaagtg cagttagggc tgggaagggt ctaccctcgg ccgccgtcca agacctaccg 60
   aggagctttc cagaatctgt tccagagcgt gcgcgaagtg atccagaacc cgggccccag 120
   gcacccagag gccgcgagcg cagcacctcc cggcgccagt ttgctgctgc tgcagcagca 180
   gcagcagcag cagcagcagc agcagcagca gcagcagcag cagcagcagc agcagcagca 240
   agagactagc cccaggcagc agcagcagca gcagggtgag gatggttctc cccaagccca 300
   tcgtagaggc cccacaggct acctggtcct ggatgaggaa cagcaacctt cacagccgca 360
   gtcggccctg gagtgccacc ccgagagagg ttgcgtccca gagcctggag ccgccgtggc 420
   cgccagcaag gggctgccgc agcagctgcc agcacctccg gacgaggatg actcagctgc 480
   cccatccacg ttgtccctgc tgggccccac tttccccggc ttaagcagct gctccgctga 540
   ccttaaagac atcctgagcg aggccagcac catgcaactc cttcagcaac agcagcagga 600
   agcagtatcc gaaggcagca gcagcgggag agcgagggag gcctcggggg ctcccacttc 660
   ctccaaggac aattacttag ggggcacttc gaccatttct gacaacgcca aggagttgtg 720
   taaggcagtg tcggtgtcca tgggcctggg tgtggaggcg ttggagcatc tgagtccagg 780
   ggaacagctt cggggggatt gcatgtacgc cccacttttg ggagttccac ccgctgtgcg 840
   tcccactcct tgtgccccat tggccgaatg caaaggttct ctgctagacg acagcgcagg 900
   caagagcact gaagatactg ctgagtattc ccctttcaag ggaggttaca ccaaagggct 960
   agaaggcgag agcctaggct gctctggcag cgctgcagca gggagctccg ggacacttga 1020
   actgccgtct accctgtctc tctacaagtc cggagcactg gacgaggcag ctgcgtacca 1080
   gagtcgcgac tactacaact ttccactggc tctggccgga ccgccgcccc ctccgccgcc 1140
   tccccatccc cacgctcgca tcaagctgga gaacccgctg gactacggca gcgcctgggc 1200
   ggctgcggcg gcgcagtgcc gctatgggga cctggcgagc ctgcatggcg cgggtgcagc 1260
   gggacccggt tctgggtcac cctcagccgc cgcttcctca tcctggcaca ctctcttcac 1320
   agccgaagaa ggccagttgt atggaccgtg tggtggtggt gggggtggtg gcggcggcgg 1380
   cggcggcggc ggcggcggcg gcggcggcgg cggcggcggc gaggcgggag ctgtagcccc 1440
   ctacggctac actcggcccc ctcaggggct ggcgggccag gaaagcgact tcaccgcacc 1500
   tgatgtgtgg taccctggcg gcatggtgag cagagtgccc tatcccagtc ccacttgtgt 1560
   caaaagcgaa atgggcccct ggatggatag ctactccgga ccttacgggg acatgcgttt 1620
   ggagactgcc agggaccatg ttttgcccat tgactattac tttccacccc agaagacctg 1680
   cctgatctgt ggagatgaag cttctgggtg tcactatgga gctctcacat gtggaagctg 1740
   caaggtcttc ttcaaaagag ccgctgaagg gaaacagaag tacctgtgcg ccagcagaaa 1800
   tgattgcact attgataaat tccgaaggaa aaattgtcca tcttgtcgtc ttcggaaatg 1860
   ttatgaagca gggatgactc tgggagacaa cttacctgag caagctgctt tttggagaca 1920
   tttgcacatc ttttgggatc acgttgttaa gaagtag 1957
<210> 15
   <211> 668
   <212> PRT
   <213> Homo sapiens
<400> 15
   Met Glu Val Gln Leu Gly Leu Gly Arg Val Tyr Pro Arg Pro Pro Ser
   1               5                   10                  15
   Lys Thr Tyr Arg Gly Ala Phe Gln Asn Leu Phe Gln Ser Val Arg Glu
               20                  25                  30
   Val Ile Gln Asn Pro Gly Pro Arg His Pro Glu Ala Ala Ser Ala Ala
           35                  40                  45
   Pro Pro Gly Ala Ser Leu Leu Leu Gln Gln Gln Gln Gln Gln Gln Gln
       50                  55                  60
   Gln Gln Gln Gln Gln Gln Gln Gln Gln Gln Gln Gln Gln Gln Gln Gln
   65                  70                  75                  80
   Gln Gln Gln Gln Gln Glu Thr Ser Pro Arg Gln Gln Gln Gln Gln Gln
                   85                  90                  95
   Gly Glu Asp Gly Ser Pro Gln Ala His Arg Arg Gly Pro Thr Gly Tyr
               100                 105                 110
   Leu Val Leu Asp Glu Glu Gln Gln Pro Ser Gln Pro Gln Ser Ala Leu
           115                 120                 125
   Glu Cys His Pro Glu Arg Gly Cys Val Pro Glu Pro Gly Ala Ala Val
       130                 135                 140
   Ala Ala Ser Lys Gly Leu Pro Gln Gln Leu Pro Ala Pro Pro Asp Glu
   145                 150                 155                 160
   Asp Asp Ser Ala Ala Pro Ser Thr Leu Ser Leu Leu Gly Pro Thr Phe
                   165                 170                 175
   Pro Gly Leu Ser Ser Cys Ser Ala Asp Leu Lys Asp Ile Leu Ser Glu
               180                 185                 190
   Ala Ser Thr Met Gln Leu Leu Gln Gln Gln Gln Gln Glu Ala Val Ser
           195                 200                 205
   Glu Gly Ser Ser Ser Gly Arg Ala Arg Glu Ala Ser Gly Ala Pro Thr
       210                 215                 220
   Ser Ser Lys Asp Asn Tyr Leu Gly Gly Thr Ser Thr Ile Ser Asp Asn
   225                 230                 235                 240
   Ala Lys Glu Leu Cys Lys Ala Val Ser Val Ser Met Gly Leu Gly Val
                   245                 250                 255
   Glu Ala Leu Glu His Leu Ser Pro Gly Glu Gln Leu Arg Gly Asp Cys
               260                 265                 270
   Met Tyr Ala Pro Leu Leu Gly Val Pro Pro Ala Val Arg Pro Thr Pro
           275                 280                 285
   Cys Ala Pro Leu Ala Glu Cys Lys Gly Ser Leu Leu Asp Asp Ser Ala
       290                 295                 300
   Gly Lys Ser Thr Glu Asp Thr Ala Glu Tyr Ser Pro Phe Lys Gly Gly
   305                 310                 315                 320
   Tyr Thr Lys Gly Leu Glu Gly Glu Ser Leu Gly Cys Ser Gly Ser Ala
                   325                 330                 335
   Ala Ala Gly Ser Ser Gly Thr Leu Glu Leu Pro Ser Thr Leu Ser Leu
               340                 345                 350
   Tyr Lys Ser Gly Ala Leu Asp Glu Ala Ala Ala Tyr Gln Ser Arg Asp
           355                 360                 365
   Tyr Tyr Asn Phe Pro Leu Ala Leu Ala Gly Pro Pro Pro Pro Pro Pro
       370                 375                 380
   Pro Pro His Pro His Ala Arg Ile Lys Leu Glu Asn Pro Leu Asp Tyr
   385                 390                 395                 400
   Gly Ser Ala Trp Ala Ala Ala Ala Ala Gln Cys Arg Tyr Gly Asp Leu
                   405                 410                 415
   Ala Ser Leu His Gly Ala Gly Ala Ala Gly Pro Gly Ser Gly Ser Pro
               420                 425                 430
   Ser Ala Ala Ala Ser Ser Ser Trp His Thr Leu Phe Thr Ala Glu Glu
           435                 440                 445
   Gly Gln Leu Tyr Gly Pro Cys Gly Gly Gly Gly Gly Gly Gly Gly Gly
       450                 455                 460
   Gly Gly Gly Gly Gly Gly Gly Gly Gly Gly Glu Ala Gly Ala Val Ala
   465                 470                 475                 480
   Pro Tyr Gly Tyr Thr Arg Pro Pro Gln Gly Leu Ala Gly Gln Glu Ser
                   485                 490                 495
   Asp Phe Thr Ala Pro Asp Val Trp Tyr Pro Gly Gly Met Val Ser Arg
               500                 505                 510
   Val Pro Tyr Pro Ser Pro Thr Cys Val Lys Ser Glu Met Gly Pro Trp
           515                 520                 525
   Met Asp Ser Tyr Ser Gly Pro Tyr Gly Asp Met Arg Leu Glu Thr Ala
       530                 535                 540
   Arg Asp His Val Leu Pro Ile Asp Tyr Tyr Phe Pro Pro Gln Lys Thr
   545                 550                 555                 560
   Cys Leu Ile Cys Gly Asp Glu Ala Ser Gly Cys His Tyr Gly Ala Leu
                   565                 570                 575
   Thr Cys Gly Ser Cys Lys Val Phe Phe Lys Arg Ala Ala Glu Gly Lys
               580                 585                 590
   Gln Lys Tyr Leu Cys Ala Ser Arg Asn Asp Cys Thr Ile Asp Lys Phe
           595                 600                 605
   Arg Arg Lys Asn Cys Pro Ser Cys Arg Leu Arg Lys Cys Tyr Glu Ala
       610                 615                 620
   Gly Met Thr Leu Gly Ala Ser Ser Gly Thr Asn Ser Val Phe Leu Pro
   625                 630                 635                 640
   His Arg Asp Val Val Arg Thr Gly Cys Arg Ser Asn Ser Gly Tyr His
                   645                 650                 655
   Ser Cys Ser Cys Glu Tyr His Asp Tyr Cys Phe Leu
               660                 665
<210> 16
   <211> 2010
   <212> DNA
   <213> Homo sapiens
<400> 16
   gatggaagtg cagttagggc tgggaagggt ctaccctcgg ccgccgtcca agacctaccg      60
   aggagctttc cagaatctgt tccagagcgt gcgcgaagtg atccagaacc cgggccccag     120
   gcacccagag gccgcgagcg cagcacctcc cggcgccagt ttgctgctgc agcagcagca     180
   gcagcagcag cagcagcagc agcagcagca gcagcagcag cagcagcagc agcagcagca     240
   gcagcagcag cagcaagaga ctagccccag gcagcagcag cagcagcagg gtgaggatgg     300
   ttctccccaa gcccatcgta gaggccccac aggctacctg gtcctggatg aggaacagca     360
   accttcacag ccgcagtcgg ccctggagtg ccaccccgag agaggttgcg tcccagagcc     420
   tggagccgcc gtggccgcca gcaaggggct gccgcagcag ctgccagcac ctccggacga     480
   ggatgactca gctgccccat ccacgttgtc cctgctgggc cccactttcc ccggcttaag     540
   cagctgctcc gctgacctta aagacatcct gagcgaggcc agcaccatgc aactccttca     600
   gcaacagcag caggaagcag tatccgaagg cagcagcagc gggagagcga gggaggcctc     660
   gggggctccc acttcctcca aggacaatta cttagggggc acttcgacca tttctgacaa     720
   cgccaaggag ttgtgtaagg cagtgtcggt gtccatgggc ctgggtgtgg aggcgttgga     780
   gcatctgagt ccaggggaac agcttcgggg ggattgcatg tacgccccac ttttgggagt     840
   tccacccgct gtgcgtccca ctccttgtgc cccattggcc gaatgcaaag gttctctgct     900
   agacgacagc gcaggcaaga gcactgaaga tactgctgag tattcccctt tcaagggagg     960
   ttacaccaaa gggctagaag gcgagagcct aggctgctct ggcagcgctg cagcagggag     1020
   ctccgggaca cttgaactgc cgtctaccct gtctctctac aagtccggag cactggacga     1080
   ggcagctgcg taccagagtc gcgactacta caactttcca ctggctctgg ccggaccgcc     1140
   gccccctccg ccgcctcccc atccccacgc tcgcatcaag ctggagaacc cgctggacta     1200
   cggcagcgcc tgggcggctg cggcggcgca gtgccgctat ggggacctgg cgagcctgca     1260
   tggcgcgggt gcagcgggac ccggttctgg gtcaccctca gccgccgctt cctcatcctg     1320
   gcacactctc ttcacagccg aagaaggcca gttgtatgga ccgtgtggtg gtggtggggg     1380
   tggtggcggc ggcggcggcg gcggcggcgg cggcggcggc ggcgaggcgg gagctgtagc     1440
   cccctacggc tacactcggc cccctcaggg gctggcgggc caggaaagcg acttcaccgc     1500
   acctgatgtg tggtaccctg gcggcatggt gagcagagtg ccctatccca gtcccacttg     1560
   tgtcaaaagc gaaatgggcc cctggatgga tagctactcc ggaccttacg gggacatgcg     1620
   tttggagact gccagggacc atgttttgcc cattgactat tactttccac cccagaagac     1680
   ctgcctgatc tgtggagatg aagcttctgg gtgtcactat ggagctctca catgtggaag     1740
   ctgcaaggtc ttcttcaaaa gagccgctga agggaaacag aagtacctgt gcgccagcag     1800
   aaatgattgc actattgata aattccgaag gaaaaattgt ccatcttgtc gtcttcggaa     1860
   atgttatgaa gcagggatga ctctgggagc ctccagcggg accaatagtg ttttcctacc     1920
   tcacagggat gttgtgagga cgggctgtag aagtaatagt ggttaccatt catgtagttg     1980
   tgagtatcat gattattgtt tcctgtaatg                                      2010
<210> 17
   <211> 388
   <212> PRT
   <213> Homo sapiens
<400> 17
   Met Ile Leu Trp Leu His Ser Leu Glu Thr Ala Arg Asp His Val Leu
   1               5                   10                  15
   Pro Ile Asp Tyr Tyr Phe Pro Pro Gln Lys Thr Cys Leu Ile Cys Gly
               20                  25                  30
   Asp Glu Ala Ser Gly Cys His Tyr Gly Ala Leu Thr Cys Gly Ser Cys
           35                  40                  45
   Lys Val Phe Phe Lys Arg Ala Ala Glu Gly Lys Gln Lys Tyr Leu Cys
       50                  55                  60
   Ala Ser Arg Asn Asp Cys Thr Ile Asp Lys Phe Arg Arg Lys Asn Cys
   65                  70                  75                  80
   Pro Ser Cys Arg Leu Arg Lys Cys Tyr Glu Ala Gly Met Thr Leu Gly
                   85                  90                  95
   Ala Arg Lys Leu Lys Lys Leu Gly Asn Leu Lys Leu Gln Glu Glu Gly
               100                 105                 110
   Glu Ala Ser Ser Thr Thr Ser Pro Thr Glu Glu Thr Thr Gln Lys Leu
           115                 120                 125
   Thr Val Ser His Ile Glu Gly Tyr Glu Cys Gln Pro Ile Phe Leu Asn
       130                 135                 140
   Val Leu Glu Ala Ile Glu Pro Gly Val Val Cys Ala Gly His Asp Asn
   145                 150                 155                 160
   Asn Gln Pro Asp Ser Phe Ala Ala Leu Leu Ser Ser Leu Asn Glu Leu
                   165                 170                 175
   Gly Glu Arg Gln Leu Val His Val Val Lys Trp Ala Lys Ala Leu Pro
               180                 185                 190
   Gly Phe Arg Asn Leu His Val Asp Asp Gln Met Ala Val Ile Gln Tyr
           195                 200                 205
   Ser Trp Met Gly Leu Met Val Phe Ala Met Gly Trp Arg Ser Phe Thr
       210                 215                 220
   Asn Val Asn Ser Arg Met Leu Tyr Phe Ala Pro Asp Leu Val Phe Asn
   225                 230                 235                 240
   Glu Tyr Arg Met His Lys Ser Arg Met Tyr Ser Gln Cys Val Arg Met
                   245                 250                 255
   Arg His Leu Ser Gln Glu Phe Gly Trp Leu Gln Ile Thr Pro Gln Glu
               260                 265                 270
   Phe Leu Cys Met Lys Ala Leu Leu Leu Phe Ser Ile Ile Pro Val Asp
           275                 280                 285
   Gly Leu Lys Asn Gln Lys Phe Phe Asp Glu Leu Arg Met Asn Tyr Ile
       290                 295                 300
   Lys Glu Leu Asp Arg Ile Ile Ala Cys Lys Arg Lys Asn Pro Thr Ser
   305                 310                 315                 320
   Cys Ser Arg Arg Phe Tyr Gln Leu Thr Lys Leu Leu Asp Ser Val Gln
                   325                 330                 335
   Pro Ile Ala Arg Glu Leu His Gln Phe Thr Phe Asp Leu Leu Ile Lys
               340                 345                 350
   Ser His Met Val Ser Val Asp Phe Pro Glu Met Met Ala Glu Ile Ile
           355                 360                 365
   Ser Val Gln Val Pro Lys Ile Leu Ser Gly Lys Val Lys Pro Ile Tyr
       370                 375                 380
   Phe His Thr Gln
   385
<210> 18
   <211> 1169
   <212> DNA
   <213> Homo sapiens
<400> 18
   atgatactct ggcttcacag tttggagact gccagggacc atgttttgcc cattgactat 60
   tactttccac cccagaagac ctgcctgatc tgtggagatg aagcttctgg gtgtcactat 120
   ggagctctca catgtggaag ctgcaaggtc ttcttcaaaa gagccgctga agggaaacag 180
   aagtacctgt gcgccagcag aaatgattgc actattgata aattccgaag gaaaaattgt 240
   ccatcttgtc gtcttcggaa atgttatgaa gcagggatga ctctgggagc ccggaagctg 300
   aagaaacttg gtaatctgaa actacaggag gaaggagagg cttccagcac caccagcccc 360
   actgaggaga caacccagaa gctgacagtg tcacacattg aaggctatga atgtcagccc 420
   atctttctga atgtcctgga agccattgag ccaggtgtag tgtgtgctgg acacgacaac 480
   aaccagcccg actcctttgc agccttgctc tctagcctca atgaactggg agagagacag 540
   cttgtacacg tggtcaagtg ggccaaggcc ttgcctggct tccgcaactt acacgtggac 600
   gaccagatgg ctgtcattca gtactcctgg atggggctca tggtgtttgc catgggctgg 660
   cgatccttca ccaatgtcaa ctccaggatg ctctacttcg cccctgatct ggttttcaat 720
   gagtaccgca tgcacaagtc ccggatgtac agccagtgtg tccgaatgag gcacctctct 780
   caagagtttg gatggctcca aatcaccccc caggaattcc tgtgcatgaa agcactgcta 840
   ctcttcagca ttattccagt ggatgggctg aaaaatcaaa aattctttga tgaacttcga 900
   atgaactaca tcaaggaact cgatcgtatc attgcatgca aaagaaaaaa tcccacatcc 960
   tgctcaagac gcttctacca gctcaccaag ctcctggact ccgtgcagcc tattgcgaga 1020
   gagctgcatc agttcacttt tgacctgcta atcaagtcac acatggtgag cgtggacttt 1080
   ccggaaatga tggcagagat catctctgtg caagtgccca agatcctttc tgggaaagtc 1140
   aagcccatct atttccacac ccagtgaag 1169
<210> 19
   <211> 124
   <212> DNA
   <213> Homo sapiens
<400> 19
   gtcgtcttcg gaaatgttat gaagcaggga tgactctggg agcagctgtt gttgtttctg 60
   aaagaatctt gagggtgttt ggagtctcag aatggcttcc ttaaagacta ccttcagact 120
   ctca 124
<210> 20
   <211> 141
   <212> DNA
   <213> Homo sapiens
<400> 20
   ctgggaggga aacagaagta cctgtgcgcc agcagaaatg attgcactat tgataaattc     60
   cgaaggaaaa attgtccatc ttgtcgtctt cggaaatgtt atgaagcagg gatgactctg    120
   ggagcagctg ttgttgtttc t                                              141
<210> 21
   <211> 119
   <212> DNA
   <213> Homo sapiens
<400> 21
   cttctgggtg tcactatgga gctctcacat gtggaagctg caaggtcttc ttcaaaagag     60
   ccgctgaagg atttttcaga atgaacaaat taaaagaatc atcagacact aaccccaag     119
<210> 22
   <211> 131
   <212> DNA
   <213> Homo sapiens
<400> 22
   cttgtcgtct tcggaaatgt tatgaagcag ggatgactct gggagactag aattccaaag     60
   accctcaggc tggtgatgca agtgggaagt ctcatttctg agaagtgctg cttcctaccc    120
   acaattcttt g                                                         131
<210> 23
   <211> 117
   <212> DNA
   <213> Homo sapiens
<400> 23
   gtccatcttg tcgtcttcgg aaatgttatg aagcagggat gactctggga gaaaaattcc     60
   gggttggcaa ttgcaagcat ctcaaaatga ccagaccctg aagaaaggct gacttgc       117
<210> 24
   <211> 131
   <212> DNA
   <213> Homo sapiens
<400> 24
   gtcttcggaa atgttatgaa gcagggatga ctctgggaga caacttacct gagcaagctg     60
   ctttttggag acatttgcac atcttttggg atcacgttgt taagaagtag aactaaggga    120
   aaaacacgca g                                                         131
<210> 25
   <211> 156
   <212> DNA
   <213> Homo sapiens
<400> 25
   ttgtccatct tgtcgtcttc ggaaatgtta tgaagcaggg atgactctgg gagcctccag     60
   cgggaccaat agtgttttcc tacctcacag ggatgttgtg aggacgggct gtagaagtaa    120
   tagtggttac cattcatgta gttgtgagta tcatga                              156
<210> 26
   <211> 121
   <212> DNA
   <213> Homo sapiens
<400> 26
   ctgtcacttt ttcccatgat actctggctt cacagtttgg agactgccag ggaccatgtt     60
   ttgcccattg actattactt tccaccccag aagacctgcc tgatctgtgg agatgaagct    120
   t                                                                    121
<210> 27
   <211> 19
   <212> DNA
   <213> Artificial Sequence
<220>
   <223> primer
<400> 27
   gtcgtcttcg gaaatgtta 19
<210> 28
   <211> 20
   <212> DNA
   <213> Artificial Sequence
<220>
   <223> primer
<400> 28
   tgagagtctg aaggtagtct 20
<210> 29
   <211> 21
   <212> DNA
   <213> Artificial Sequence
<220>
   <223> probe
<400> 29
   actctgggag cagctgttgt t 21
<210> 30
   <211> 18
   <212> DNA
   <213> Artificial Sequence
<220>
   <223> primer
<400> 30
   ctgggaggga aacagaag 18
<210> 31
   <211> 19
   <212> DNA
   <213> Artificial Sequence
<220>
   <223> primer
<400> 31
   agaaacaaca acagctgct 19
<210> 32
   <211> 21
   <212> DNA
   <213> Artificial Sequence
<220>
   <223> probe
<400> 32
   tgtgcgccag cagaaatgat t 21
<210> 33
   <211> 18
   <212> DNA
   <213> Artificial Sequence
<220>
   <223> primer
<400> 33
   cttctgggtg tcactatg 18
<210> 34
   <211> 19
   <212> DNA
   <213> Artificial Sequence
<220>
   <223> primer
<400> 34
   cttggggtta gtgtctgat 19
<210> 35
   <211> 24
   <212> DNA
   <213> Artificial Sequence
<220>
   <223> probe
<400> 35
   agagccgctg aaggattttt caga 24
<210> 36
   <211> 19
   <212> DNA
   <213> Artificial Sequence
<220>
   <223> primer
<400> 36
   tcttgtcgtc ttcggaaat 19
<210> 37
   <211> 19
   <212> DNA
   <213> Artificial Sequence
<220>
   <223> primer
<400> 37
   cttggggtta gtgtctgat 19
<210> 38
   <211> 24
   <212> DNA
   <213> Artificial Sequence
<220>
   <223> probe
<400> 38
   tgactctggg aggatttttc agaa 24
<210> 39
   <211> 18
   <212> DNA
   <213> Artificial Sequence
<220>
   <223> primer
<400> 39
   cttgtcgtct tcggaaat 18
<210> 40
   <211> 24
   <212> DNA
   <213> Artificial Sequence
<220>
   <223> primer
<400> 40
   caaagaattg tgggtaggaa gcag 24
<210> 41
   <211> 24
   <212> DNA
   <213> Artificial Sequence
<220>
   <223> probe
<400> 41
   agggatgact ctgggagact agaa 24
<210> 42
   <211> 17
   <212> DNA
   <213> Artificial Sequence
<220>
   <223> primer
<400> 42
   ggatgactct gggagca 17
<210> 43
   <211> 19
   <212> DNA
   <213> Artificial Sequence
<220>
   <223> primer
<400> 43
   cctgagggtc tttggaatt 19
<210> 44
   <211> 24
   <212> DNA
   <213> Artificial Sequence
<220>
   <223> probe
<400> 44
   aacgtggaac tcagttactg ggat 24
<210> 45
   <211> 18
   <212> DNA
   <213> Artificial Sequence
<220>
   <223> primer
<400> 45
   gtccatcttg tcgtcttc 18
<210> 46
   <211> 19
   <212> DNA
   <213> Artificial Sequence
<220>
   <223> primer
<400> 46
   gcaagtcagc ctttcttca 19
<210> 47
   <211> 22
   <212> DNA
   <213> Artificial Sequence
<220>
   <223> probe
<400> 47
   gggagaaaaa ttccgggttg gc 22
<210> 48
   <211> 19
   <212> DNA
   <213> Artificial Sequence
<220>
   <223> primer
<400> 48
   cgaaataccc gaagaaaga 19
<210> 49
   <211> 19
   <212> DNA
   <213> Artificial Sequence
<220>
   <223> primer
<400> 49
   ggacaatttt tccttcgga 19
<210> 50
   <211> 23
   <212> DNA
   <213> Artificial Sequence
<220>
   <223> probe
<400> 50
   tgttctccca gggaaacaga agt 23
<210> 51
   <211> 20
   <212> DNA
   <213> Artificial Sequence
<220>
   <223> primer
<400> 51
   gtcttcggaa atgttatgaa 20
<210> 52
   <211> 19
   <212> DNA
   <213> Artificial Sequence
<220>
   <223> primer
<400> 52
   ctgcgtgttt ttcccttag 19
<210> 53
   <211> 23
   <212> DNA
   <213> Artificial Sequence
<220>
   <223> probe
<400> 53
   tctgggagac aacttacctg agc 23
<210> 54
   <211> 18
   <212> DNA
   <213> Artificial Sequence
<220>
   <223> primer
<400> 54
   ttgtccatct tgtcgtct 18
<210> 55
   <211> 19
   <212> DNA
   <213> Artificial Sequence
<220>
   <223> primer
<400> 55
   tcatgatact cacaactac 19
<210> 56
   <211> 20
   <212> DNA
   <213> Artificial Sequence
<220>
   <223> probe
<400> 56
   tacctcacag ggatgttgtg 20
<210> 57
   <211> 18
   <212> DNA
   <213> Artificial Sequence
<220>
   <223> primer
<400> 57
   agagagacag cttgtaca 18
<210> 58
   <211> 18
   <212> DNA
   <213> Artificial Sequence
<220>
   <223> primer
<400> 58
   ccatcatttc cggaaagt 18
<210> 59
   <211> 22
   <212> DNA
   <213> Artificial Sequence
<220>
   <223> probe
<400> 59
   cttgcctgat tgcgagagag ct 22
<210> 60
   <211> 18
   <212> DNA
   <213> Artificial Sequence
<220>
   <223> primer
<400> 60
   ggaattcctg tgcatgaa 18
<210> 61
   <211> 20
   <212> DNA
   <213> Artificial Sequence
<220>
   <223> primer
<400> 61
   gcaactacta gagaaaagga 20
<210> 62
   <211> 25
   <212> DNA
   <213> Artificial Sequence
<220>
   <223> probe
<400> 62
   cagcattaga ttatgggtcc ttcac 25
<210> 63
   <211> 25
   <212> DNA
   <213> Artificial Sequence
<220>
   <223> primer
<400> 63
   catcctgctc aagacgcttc tacca 25
<210> 64
   <211> 20
   <212> DNA
   <213> Artificial Sequence
<220>
   <223> primer
<400> 64
   gcaactacta gagaaaagga 20
<210> 65
   <211> 20
   <212> DNA
   <213> Artificial Sequence
<220>
   <223> probe
<400> 65
   gtgcagcctg attatgggtc 20
<210> 66
   <211> 18
   <212> DNA
   <213> Artificial Sequence
<220>
   <223> primer
<400> 66
   ctgctcaaga cgcttcta 18
<210> 67
   <211> 19
   <212> DNA
   <213> Artificial Sequence
<220>
   <223> primer
<400> 67
   atcatttccg gaaagtcca 19
<210> 68
   <211> 20
   <212> DNA
   <213> Artificial Sequence
<220>
   <223> probe
<400> 68
   tccgtgcagc ctattgcgag 20
<210> 69
   <211> 19
   <212> DNA
   <213> Artificial Sequence
<220>
   <223> primer
<400> 69
   ctgtcacttt ttcccatga 19
<210> 70
   <211> 18
   <212> DNA
   <213> Artificial Sequence
<220>
   <223> primer
<400> 70
   aagcttcatc tccacaga 18
<210> 71
   <211> 22
   <212> DNA
   <213> Artificial Sequence
<220>
   <223> probe
<400> 71
   tggcttcaca gtttggagac tg 22